(19) **Europäisches Patentamt / European Patent Office / Office européen des brevets**

(11) **EP 3 600 224 B1**

(12) **EUROPEAN PATENT SPECIFICATION**

(45) Date of publication and mention
of the grant of the patent:
**06.05.2026 Bulletin 2026/19**

(21) Application number: **18771644.4**

(22) Date of filing: **23.03.2018**

(51) International Patent Classification (IPC):
**A61K 8/25** *(2006.01)*    **A61K 8/81** *(2006.01)*
**A61Q 19/00** *(2006.01)*    **A61Q 17/04** *(2006.01)*

(52) Cooperative Patent Classification (CPC):
**A61K 8/8182; A61Q 19/00;** A61K 2800/33;
A61Q 17/04

(86) International application number:
**PCT/US2018/023931**

(87) International publication number:
**WO 2018/175836 (27.09.2018 Gazette 2018/39)**

(54) **SKIN CARE COMPOSITIONS COMPRISING A TERPOLYMER, PROCESS FOR PREPARING THE SAME AND METHOD OF USE THEREOF**

HAUTPFLEGEZUSAMMENSETZUNGEN MIT EINEM TERPOLYMER, VERFAHREN ZUR HERSTELLUNG DAVON UND VERFAHREN ZUR VERWENDUNG DAVON

COMPOSITIONS DE SOIN DE LA PEAU COMPRENANT UN TERPOLYMÈRE ET PROCESSUS DE PRÉPARATION ET PROCÉDÉ D'UTILISATION DE CES DERNIÈRES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2017 US 201762475291 P**

(43) Date of publication of application:
**05.02.2020 Bulletin 2020/06**

(73) Proprietor: **ISP Investments LLC
Wilmington, DE 19805 (US)**

(72) Inventors:
• **FARES, Hani M.
Somerset
New Jersey 08873 (US)**
• **KENNEDY, Diane M.
Bayonne
New Jersey 07002 (US)**
• **PRETTYPAUL, Donald I.
Englewood
New Jersey 07631 (US)**
• **GUERRERO, Ritamarie
Hillsborough
New Jersey 08844 (US)**

(74) Representative: **Kutzenberger Wolff & Partner
Waidmarkt 11
50676 Köln (DE)**

(56) References cited:
WO-A1-2006/097514    WO-A1-2015/116899
WO-A1-2015/153461    WO-A2-2009/023662
US-A- 5 686 067    US-A1- 2007 231 355
US-A1- 2012 219 515    US-B1- 6 294 158
US-B1- 6 436 377

**Description**

**FIELD OF THE INVENTION**

The present application relates to a skin care composition, and, more particularly, to a sunscreen composition wherein the skin care and the sunscreen compositions demonstrate enhanced water resistance, and provide dry and powdery feel and decreased shine appearance on the surface of the skin after its application.

**BACKGROUND OF THE INVENTION**

Water resistance property is sought as one of the most desirable properties in the cosmetic/personal care industry. There have been considerable efforts in the cosmetic/personal care industry to provide personal care/cosmetic products having improved water resistance property, more particularly for sunscreen products, skin care products, and other cosmetic products. Water resistance property of the skin care products especially of the sunscreen products is considered as one of the key determinants in addition to the other critical elements such as sun protection factor (SPF) and UVA protection. It is a well-known approach to use the sunscreen products to limit the Ultra Violet (UV) radiation exposure to the skin, and it has been observed that most of the commercially available sunscreen/skin care products suffer from poor water resistance property, and the main reason for this poor water resistance property of these products is presence of surfactants. Most of these skin care products are available as emulsions comprising surfactants to reduce the surface tension between the hydrophilic and the hydrophobic ingredients of these products, however, use of such surfactants disadvantageously results in decreased water resistance of these products. Due to this decreased water resistance property of these products, they are tending to remove easily on exposure to water or sweat, thus, necessities multiple applications of skin care products.

United States Patent Publication No.2012195839 discloses an aqueous volatile solvent-based composition comprising at least one UV absorbing active ingredient and at least one film forming polymer containing a plurality of acid groups, wherein the polymer is present in an amount greater than about 1% by weight of the composition and wherein at least a portion of the acid groups have been neutralized with a neutralizing agent. The disclosed compositions are for topical application for example, skin and hair, for protection against UV radiations.

PCT Publication No. 2009023662 discloses a water-resistant, rub-resistant, sprayable, clear, homogeneous sunscreen composition comprising: (a) an active sunscreen ingredient; (b) alcohol; and (c) a vinyl lactam or maleimide polymer soluble in said composition.

United States Patent No. 6294158 discloses a cosmetic composition for the treatment of keratinous material, comprising, in a cosmetically acceptable aqueous medium, at least one anionic polymer and an acrylic terpolymer comprising: (a) about 20 to 70% by weight, of a carboxylic acid containing $\alpha,\beta$-monoethylenic unsaturation; (b) about 20 to 80% by weight, of a non-surfactant monomer containing monoethylenic unsaturation, which is different from (a); and (c) about 0.5 to 60% by weight, of a nonionic urethane monomer which is the product of reaction of a monohydric nonionic surfactant with a monoisocyanate containing monoethylenic unsaturation.

In spite of continuous efforts in the personal care/cosmetic industry to develop skin care and/or sunscreen products having improved water resistance properties, there is still a need to envisage skin care compositions comprising (i) improved water resistance property, (ii) dry and powdery feel; and (iii) decreased shine appearance on the surface of the skin. Accordingly, it is an objective of the present application to provide a skin care composition having said properties. See also prior art documents WO 2009/023662 A2 and WO 2006/097514 A1.

**SUMMARY OF THE INVENTION**

The present application provides a skin care composition according to claim 1.

Another aspect of the present application provides a sunscreen composition according to claim 3.

According to another important embodiment of the present application, the weight average molecular weight of terpolymer employed in skincare and sunscreen composition would include from 150,000 to 300,000 Daltons.

According to another embodiment of the present application discloses to employ at least one cosmetically/-dermatologically acceptable additive agents for preparing skin care and sunscreen composition of the present application, and wherein, the cosmetically/dermatologically acceptable additives would include but not limited to additive diluents, emollients, humectants, thickeners, preservatives, coloring agents, sunscreen agents, moisturizing agents, film formers/waterproofing agents, bio-active ingredients, pharmaceutical ingredients, pH adjusting agents, chelating agents, rheology modifying agents, fragrance agents, plant extracts, absorbents, vitamins, photo stabilizing agents, sunscreen boosters, anti-oxidants, exfoliating agents, liquid carriers, waxes, conditioners, lubricants, anti-bacterial agents, anti-aging agents, and various combinations thereof.

In accordance with another embodiment of the present application, the skin care and the sunscreen compositions of the present application exhibits enhanced water resistance, and provides dry and powdery feel and decreased shine appearance to the skin after its application.

Yet another embodiment of the present application discloses that the skin care and the sunscreen compositions of the present application can be formulated in the form of a gel, a balm, a cream-in-gel, a balm-in-gel, a stick, a lotion, a foam, a foam cream, a cream, a moisturizer, a spray, an ointment, and a lamellar gel, and wherein the pH range of these compositions are in the range of from about 2 to about 12.

## BRIEF DESCRIPTION OF THE FIGURES

Further embodiments of the present invention can be understood with the appended figures.

FIG. 1 illustrates comparative study of the water resistance property of the skin care composition of Example 1a in comparison with skin care compositions of Example 1b and Example 1c.

FIG. 2 illustrates comparative study of the water resistance property of the skin care composition of Example 3a in comparison with skin care compositions of Example 3b and Example 3c.

FIG. 3 illustrates comparative study of the water resistance property of the skin care composition of Example 4a in comparison with skin care composition of Example 4b.

FIG. 4 illustrates comparative study of the water resistance property of the skin care composition of Example 6a in comparison with skin care composition of Example 6b.

FIG. 5 illustrates comparative study of the water resistance property of the skin care composition of Example 7a in comparison with skin care composition of Example 7b.

## DETAILED DESCRIPTION OF THE INVENTION

While this specification concludes with claims particularly pointing out and distinctly claiming that which is regarded as the invention, it is anticipated that the invention can be more readily understood through reading the following detailed description of the invention and study of the included examples

The singular forms "a," "an," and "the" include plural forms unless the context clearly dictates otherwise specified or clearly implied to the contrary by the context in which the reference is made. The term "Comprising" and "Comprises of" includes the more restrictive claims such as "Consisting essentially of" and "Consisting of".

The term "about" can indicate a difference of 10 percent of the value specified. Numerical ranges as used herein are meant to include every number and subset of numbers enclosed within that range, whether particularly disclosed or not. Further, these numerical ranges should be construed as providing support for a claim directed to any number or subset of numbers in that range.

All percentages, parts, proportions and ratios as used herein, are by weight of the total composition, unless otherwise specified. All such weights as they pertain to listed ingredients are based on the active level and, therefore; do not include solvents or byproducts that may be included in commercially available materials, unless otherwise specified.

All references to singular characteristics or limitations of the present invention shall include the corresponding plural characteristic or limitation, and *vice-versa,* unless otherwise specified or clearly implied to the contrary by the context in which the reference is made.

As used herein, the words "preferred" or "preferably" and variants refer to embodiments of the invention that afford certain benefits, under certain circumstances. However, other embodiments may also be preferred, under the same or other circumstances. Furthermore, the recitation of one or more preferred embodiments does not imply that other embodiments are not useful, and is not intended to exclude other embodiments from the scope of the invention.

References herein to "one embodiment" or "one aspect" or "one version" or "one objective" of the invention include one or more such embodiment, aspect, version or objective, unless the context clearly dictates otherwise.

All publications, articles, papers, patents, patent publications, and other references cited herein are hereby incorporated herein in their entirety for all purposes to the extent consistent with the disclosure herein.

The use of the term "at least one" will be understood to include one as well as any quantity more than one, including but not limited to, 1, 2, 3, 4, 5, 10, 15, 20, 30, 40, 50, 100, etc. The term "at least one" may extend up to 100 or 1000 or more depending on the term to which it is attached. In addition, the quantities of 100/1000 are not to be considered limiting as lower or higher limits may also produce satisfactory results. In addition, the use of the term "at least one of X, Y, and Z" will be understood to include X alone, Y alone, and Z alone, as well as any combinations of X, Y, and Z. The use of ordinal number terminology (*i.e.,* "first", "second", "third", "fourth", etc.) is solely for the purpose of differentiating between two or more items and, unless otherwise stated, is not meant to imply any sequence or order or importance to one item over another or any order of addition.

As used herein, the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "or combinations thereof" as used herein refers to all permutations and combinations of the listed items preceding the term. For example, "A, BXn, BXn+1, or combinations thereof" is intended to include at least one of: A, BXn, BXn+1, ABXn, A BXn+1, BXnBXn+1, or ABXnBXn+1 and, if order is important in a particular context, also BXnA, BXn+1A, BXn+1BXn, BXn+1BXnA, BXnBXn+1A, ABXn+1BXn, BXnABXn+1, or BXn+1ABXn. Continuing with this example, expressly included are combinations that contain repeats of one or more item or term, such as BXnBXn, AAA, MBXn, BXnBXnBXn+1, AAABXnBXn+1BXn+1BXn+1BXn+1, BXn+1BXnBXnAAA, BXn+1A BXnABXnBXn, and so forth. The skilled artisan will understand that typically there is no limit on the number of items or terms in any combination, unless otherwise apparent from the context.

The term "polymer" refers to a large molecule comprising one or more types of monomer residues (repeating units) connected by covalent chemical bonds. Polymers may be further derivatized, crosslinked, grafted or end-capped. By this definition, polymer encompasses compounds wherein the number of monomer units may range from very few, which more commonly may be called as oligomers, to very many. Non-limiting examples of polymers include homopolymers, and non-homopolymers such as copolymers, terpolymers, tetrapolymers and the higher analogues. The polymer may have a random, block, and/or alternating architecture.

The term "homopolymer" refers to a polymer that consists essentially of a single monomer type. The term "nonhomopolymer" refers to a polymer that comprises more than one monomer types. The term "copolymer" refers to a nonhomopolymer that comprises two different monomer types. The term "terpolymer" refers to a non-homopolymer that comprises three different monomer types. The term "branched" refers to any non-linear molecular structure. The term includes both branched and hyper-branched structures.

The term "keratin substrate" as used herein includes skin, nails and "keratin fibers", and wherein the "keratin fibers" means hair on head, eyelashes, eyebrows and other mammalian bodily hair.

The terms "personal care composition" and "cosmetics" refer to compositions intended for use on or in the human body, such as skin, sun, hair, oral, cosmetic, and preservative compositions, including those to alter the color and appearance of the skin and hair.

As used herein "Continuous external phase" refers to one of the phase of the system of two or more immiscible phases which is usually present in excess and makes a dispersion medium for another immiscible phase.

As used herein "Discontinuous internal phase" refers to one of the phase of the system of two or more immiscible phases which is dispersed in the continuous external phase in the form of small droplets.

The mixture of two or more immiscible phases may be prepared according to the techniques known to those skilled in the art of cosmetic and pharmaceutical arts. For example, the aqueous phase and the oil phase can be prepared separately. The aqueous phase components are mixed together under continuous stirring with heating until a uniform solution is obtained. Similarly, the oil phase components are mixed together under continuous stirring with heating until a resultant oil phase solution or dispersion is obtained. Thereafter, the oil phase solution and the aqueous phase solution admixed together under constant stirring to obtain the mixture of the two or more immiscible phases.

Further, the inventors of the present application emphasize on using the terpolymer having relative viscosity of at least at least 3.3 *cps.* The use of terpolymer having relative viscosity of 3.3 *cps* allows to reduce the level of surfactants or remove it completely from the composition. The terpolymer as used in the compositions of the present application successfully stabilizes the surface tension between the continuous external phase and the discontinuous internal phase. In accordance with one of the embodiments of the present application, the terpolymer has relative viscosity in the range of from 3.3 to 4.0 *cps.*

The relative viscosity of the terpolymer of the present application is measured through a K-value determination by capillary viscometry. In this method the relative viscosity of the 1% solution of the terpolymer is determined by dividing the flow time through a capillary viscometer of the 1% terpolymer solution, by the flow time of a 0.25N NaOH, 0.20M LiNO3 aqueous solution. K-value is calculated from the relative viscosity using the Fikentscher equation.

The relative viscosity, $\eta_{rel}$, is calculated using Equation 1

$$\eta_{rel} = \frac{\textit{flow time of solution}}{\textit{flow time of solvent}} \qquad \ldots\ldots\ldots(1)$$

The K-Value can be calculated using Equation 2

$$K = \frac{\sqrt{300c \log \eta_{rel} + (c + 1.5c \log \eta_{rel})^2} + 1.5c \log \eta_{rel} - c}{0.15c + 0.003c^2} \qquad \ldots\ldots\ldots\ldots(2)$$

According to yet another embodiment of the present application, the weight average molecular weight of the terpolymer ranges from 150,000 to 300,000 Daltons. Other weight average molecular weight of terpolymer ranges would include but not limited to from 150, 000 to 175,000 Daltons, from 175,000 to 200,000 Daltons, from 200,000 to 225,000 Daltons, from 225,000 to 250,000 Daltons, from 250,000 to 275,000 Daltons, or from 275,000 to 300, 000 Daltons. Other important and specific weight average molecular weight of terpolymer range is from 180,000 to 220,000 Daltons, and from 205,000 to 215,000 Daltons, 207,000 Daltons, 208,000 Daltons, 209,000 Daltons, 210,000 Daltons, 211,000 Daltons, or 212,000 Daltons.

One important embodiment of the present application discloses to employ 0.1 wt. % to 10 wt.% of a terpolymer of vinyl pyrrolidone/acrylic acid/lauryl methacrylate having a relative viscosity of at least 3.3 *cps,* and wherein, other ranges of terpolymer would include but not limited to 0.1 wt. % to 1 wt. %, 1 wt. % to 2 wt.%, 2 wt. % to 3 wt. %, 3 wt. % to 4 wt.%, 4 wt. % to 5 wt.%, 5 wt. % to 6 wt.%, 6 wt. % to 7 wt.%, 7 wt. % to 8 wt. %, 8 wt. % to 9 wt.%, or 9 wt. % to 10 wt.%

In another embodiment of the present application, it is contemplated that the skin care composition of the present application can be sunscreen composition, dermatological composition or a cosmetic composition.

In accordance with yet another embodiment of the present application, the skin care/sunscreen compositions of the present application can exist in the form of a gel, a balm, a cream-in-gel, a balm-in-gel, a stick, a lotion, a foam, a foam cream, a cream, a moisturizer, a spray, an ointment, and a lamellar gel. Other suitable and non-limiting sunscreen or skin care composition forms include: solutions, oils, solids, liquids, powders, gels, pastes, waxes, aerosols, mists, roll-ons, milks, emulsions, wipes, oil-in-water emulsion, water-in-oil emulsion, an oil-water-oil emulsion, a water-oil-water emulsion, a water-in-silicone emulsion, an oily solution, a lipid fusion, a hydro-alcoholic gel, an anhydrous gel, an aqueous gel, an alcoholic solutions or a hydro-alcoholic solution, suspensions, microemulsions, dispersions, microencapsulated products, tonics, mists, reconstitutable products, peels, soaps, mousses, glues, pomades, spritzes, putties, lacquers, serums, perms, powders, pencils, flakes, blush, highlighters, bronzers, concealers, and 2-way cake products.

According to another important embodiment of the present application, it is contemplated that the terpolymer of the present application can be employed in other non-limiting examples of personal care compositions that can comprise after-sun compositions, skin care compositions, oral care compositions, face care compositions, lip care compositions, eye care compositions, body care compositions, nail care compositions, anti-aging compositions, insect repellants, deodorant compositions, color cosmetic compositions, color-protection compositions, self-tanning compositions, and foot care compositions.

Example of various UV-A or UV-B based sunscreens compounds include but not limited to p-aminobenzoic acid and its derivatives, anthranilates, benzophenones, camphor derivatives, cinnamic derivatives, dibenzoyl methanes, β,β-diphenylacrylate derivatives, salicylic derivatives, triazine derivatives, benzimidazole compounds, bis-benzoazolyl derivatives, methylene bis-(hydroxyphenylbenzotriazole) compounds, sunscreen polymers, silicones, and mixtures thereof.

Examples of physical sun blockers would include but not limited to cerium oxides, chromium oxides, cobalt oxides, iron oxides, red petrolatum, silicone-treated titanium dioxides, titanium dioxides, zinc oxides, and/or zirconium oxides and mixtures thereof.

Suitable sunscreen agents for preparing the sunscreen composition of the present application would include but not limited to methoxydibenzoylmethane; octyl salicylate; pentyl dimethyl PABA; octyl dimethyl PABA; benzophenone-1; benzophenone-6; 2-(2H-benzotriazole-2-yl)-4,6-di-tert- pentylphenol; ethyl-2-cyano-3,3-diphenylacrylate; homomethyl salicylate (homosalate); bis-ethylhexyloxyphenol methoxyphenyl triazine; methyl-(l,2,2,6,6-pentamethyl-4- piperidyl)-sebacate; 2-(2H-benzotriazole-2-yl)-4-methylphenol; diethylhexyl butamido triazone; amyl dimethyl PABA; 4,6-bis(octylthiomethyl)-o-cresol; red petroleum; ethylhexyl triazone; octocrylene; isoamyl-p-methoxycinnamate; drometrizole; titanium dioxide; 2,4-di-tert-butyl-6-(5-chloro-2H-benzotriazole-2-yl)-phenol; 2-hydroxy-4- octyloxybenzophenone; benzophenone-2; diisopropyl methylcinnamate; PEG-25 PABA; 2-(l,l-dimethylethyl)-6-[[3-(l,l-demethylethyl)-2-hydroxy-5-methylphenyl]methyl-4- methylphenyl acrylate; drometrizole trisiloxane; menthyl anthranilate; butyl methoxydibenzoylmethane; 2-ethoxyethyl p-methoxycinnamate; benzylidene camphor sulfonic acid; dimethoxyphenyl-[l-(3,4)]-4,4-dimethyl 1,3-pentanedione; zinc oxide; N,N'-hexane-l,6-diylbis[3-(3,5-di-tert-butyl-4-hydroxyphenylpropionamide)]; pentaerythritol tetrakis-[3-(3,5-di-tert-butyl-4-hydroxyphenyl)propionate]; 2,6-di-tert- butyl-4-[4,6-bis(octylthio)-1,3,5-triazin-2-ylamino]phenol; 2-(2H-benzotriazole-2-yl)- 4,6-bis(1-methyl-l-phenylethyl)phenol; trolamine salicylate; diethylanolamine p- methoxycinnamate; polysilicone-15; 4-methylbenzylidene camphor; bisoctrizole; N- phenyl-benzenamine; reaction products with 2,4,4-trimethylpentene; sulisobenzone; (2- ethylhexyl)-2-cyano-3,3-diphenylacrylate; digalloyl trioleate; polyacrylamido methylbenzylidene camphor; glyceryl ethylhexanoate dimethoxycinnamate; l,3-bis-[(2'- cyano-3',3'-diphenylacryloyl)oxy]-2,2-bis-[(2'-cyano-bis-(2,2,6,6-tetramethyl-4- piperidyl) - sebacate ; benzophenone- 5 ; 1 , 3 , 5 -tris (3 ,5 -di-tert-butyl-4-hydroxybenzyl) -1,3,5- triazine-2,4,6(1H,3H,5H)-trione; hexamethylendiamine; benzophenone- 8; ethyl-4- bis(hydroxypropyl)aminobenzoate; 6-tert-butyl-2-(5-chloro-2H-benzotriazole-2-yl)-4- methylphenol; p-aminobenzoic acid; 3,3',3",5,5',5"-hexa-tert-butyl-a-a'-a"-(mesitylene- 2,4,6-triyl)tri-p-cresol; lawsone with dihydroxyacetone; benzophenone-9; benzophenone - 4; ethylhexyl dimethoxy benzylidene dioxoimidazoline propionate; N,N'-bisformyl-N,N'- bis-(2,2,6,6-tetramethyl-4-piperidinyl)-; 3-benzylidene camphor; terephthalylidene dicamphor sulfonic acid; camphor benzalkonium methosulfate; bisdisulizole disodium; etocrylene; ferulic acid; 2-(2H-benzotriazole-2-yl)-4-(l,l,3,3-tetramethylbutyl)-phenol; 4,6-bis(dodecylthiomethyl)-o-cresol; β-2-glucopyranoxy propyl hydroxy benzophenone; phenylbenzimidazole sulfonic acid; benzophenone- 3; diethylamine hydroxybenzoyl hexylbenzoate; 3',3'-diphenylacryloyloxymethyl-propane; ethylhexyl p-methoxycinnamate, and blends thereof.

According to yet another embodiment, the sunscreen compositions of present application has a sun protection factor (SPF) of at least about 10. Other non-limiting ranges of SPF of the sunscreen composition would include at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 26, at least 27, at least 28, at least 29, at least 30, at least 31, at least 32, at least 33, at least 34, at least 35, at least 36, at least 37, at least 38, at least 39, at least 40, at least 41, at least 42, at least 43, at least 43, at least 44, at least 45, at least 46, at least 47, at least 48, at least 49, at least 50, at least 51, at least 52, at least 53, at least 54, at least 55, at least 56, at least 57, at least 58, at least 59, or at least 60.

In one embodiment of the present application, at least one primary surfactant, co-surfactant or a system of surfactants comprising at least one primary surfactant and at least one co-surfactant are employed to prepare the sunscreen or skin care compositions, and wherein said primary surfactants, co-surfactants or a system surfactant can be selected from the group consisting of anionic surfactants, cationic surfactants, non-ionic surfactants, zwitterionic surfactants and amphoteric surfactants. The range of surfactants can be from 0.1 wt. % to 20 wt. % based on the total weight of the composition. Other ranges of surfactants are from 0.1 wt. % to 5 wt. %, from 5 wt. % to 10 wt. %, from 10 wt. % to 15 wt. %, or from 15 wt. % to 20 wt. % of total weight of the composition. The contemplated surfactants for use herein are as follows:

(A) Anionic Surfactants: Anionic surfactants are particularly useful in accordance with certain embodiments of the present application. Surfactants of the anionic type that may be useful include:

(1) Sulfonates and Sulfates: Suitable anionic surfactants include sulfonates and sulfates such as alkyl sulfates, alkylether sulfates, alkyl sulfonates, alkylether sulfonates, alkylbenzene sufonates, alkylbenzene ether sulfates, alkylsulfoacetates, secondary alkane sulfonates, secondary alkylsulfates, alkyl sulfosuccinates and the like. Further, examples of anionic surfactants include water-soluble salts of higher fatty acid monoglyceride monosulfates, such as the sodium salt of the monosulfated monoglyceride of hydrogenated coconut oil fatty acids, higher alkyl sulfates such as sodium lauryl sulfate, alkyl aryl sulfonates such as sodium dodecyl benzene sulfonate, higher alkyl sulfoacetates, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like.

(2) Phosphates and Phosponates: Suitable anionic surfactants also include phosphates such as alkyl phosphates, alkylether phosphates, aralkylphosphates, and aralkylether phosphates. Examples include a mixture of mono-, di- and tri-(alkyltetraglycolether)-o-phosphoric acid esters generally referred to as trilaureth-4-phosphate commercially available under the trade designation HOSTAPHAT 340KL from Clariant Corp., as well as PPG-5 ceteth 10 phosphate available under the trade designation CRODAPHOS SG from Croda Inc., Parsipanny, NJ.

(3) Amine Oxides: Suitable anionic surfactants also include amine oxides. Examples of amine oxide surfactants include lauryldimethylamine oxide, laurylamidopropyldimethylamine oxide, and/or cetyl amine oxide.

(B) Amphoteric Surfactants: Surfactants of the amphoteric type include surfactants having tertiary amine groups which may be protonated as well as quaternary amine containing zwitterionic surfactants. Those that may be useful include:

(1) Ammonium Carboxylate Amphoterics: Examples of such amphoteric surfactants include, but are not limited to: certain betaines such as cocobetaine and cocamidopropyl betaine; monoacetates such as sodium lauroamphoacetate; diacetates such as disodium lauroamphoacetate; amino- and alkylamino-propionates such as lauraminopropionic acid.

(2) Ammonium Sulfonate Amphoterics: These classes of amphoteric surfactants are often referred to as "sultaines" or "sulfobetaines" for example, cocamidopropylhydroxysultaine.

(C) Nonionic Surfactants: Surfactants of the nonionic type that may be particularly useful include:

(1) Polyethylene oxide extended sorbitan monoalkylates (*i.e.* Polysorbates); (2) Polyalkoxylated alkanols; (3) Polyalkoxylated alkylphenols include polyethoxylated octyl or nonyl phenols having HLB values of at least about 14, which are commercially available under the trade designations ICONOL and TRITON; (4) Polaxamers. Surfactants based on block copolymers of ethylene oxide (EO) and propylene oxide (PO) may also be effective. Both EO-PO-EO blocks and PO-EO-PO blocks are expected to work well as long as the HLB is at least about 14, and preferably at least about 16. Such surfactants are commercially available under the trade designations PLURONIC and TETRONIC from BASF; (5) Polyalkoxylated esters - Polyalkoxylated glycols such as ethylene glycol, propylene glycol, glycerol, and the like may be partially or completely esterified, *i.e.* one or more alcohols may be esterified, with a ($C_8$ to $C_{22}$) alkyl carboxylic acid. Such polyethoxylated esters having an HLB of at least about 14, and preferably at least about 16, may be suitable for use in compositions of the present invention; (6) Alkyl Polyglucosides - This includes glucopon 425, which has a ($C_8$ to $C_{16}$) alkyl chain length.

(D) Cationic Surfactants: Surfactants of the cationic type that may be useful include but are not limited to, primary amines, secondary amines, tertiary amines, quaternary amines, alkanolamines, mono-alkyl alkanolamines, di-alkyl alkanolamines, tri-alkyl alkanolamines, alkyl mono alkanolamines, alkyl di-alkanolamines, alkylamines, mono-alkyl amines, di-alkyl amines, tri-alkylamines, alkoxylated amines, alkyl and aryl amine alkoxylates, methoxylated alkylamines, ethoxylated alkylamines, alkoxylated alkanolamines, alkyl alkanolamines, alkoxylated ethylene diamine derivatives, alkyl/aryl/arylalkyl amine oxides. Preferred cationic surfactants of the present invention include, but are not limited to, (a) alkyl alkanolamines; and (b) alkyl tertiary amines. Additional information on useful cationic surfactants for the purpose of present invention is set forth in McCutcheon's Detergents and Emulsifiers, North American Ed., 1982 and Kirk-Othmer, Encyclopedia of Chemical Technology, 3rd Ed., Vol. 22, pp. 346-387.

Suitable emulsifiers include the following classes of ethers and esters: ethers of polyglycols and of fatty alcohols, esters of polyglycols and of fatty acids, ethers of polyglycols and of fatty alcohols which are glycosylated, esters of polyglycols and of fatty acids which are glycosylated, ethers of $C_{12-30}$ alcohols and of glycerol or of polyglycerol, esters of $C_{12-30}$ fatty acids and of glycerol or of polyglycerol, ethers of oxyalkylene-modified $C_{12-30}$ alcohols and of glycerol or polyglycerol, ethers of $C_{12-30}$ fatty alcohols comprising and of sucrose or of glucose, esters of sucrose and of $C_{12-30}$ fatty acids, esters of pentaerythritol and of $C_{12-30}$ fatty acids, esters of sorbitol and/or of sorbitan and of $C_{12-30}$ fatty acids, ethers of sorbitol and/or of sorbitan and of alkoxylated sorbitan, ethers of polyglycols and of cholesterol, esters of $C_{12-30}$ fatty acids and of alkoxylated ethers of sorbitol and/or sorbitan, and combinations thereof. Linear or branched type silicone emulsifiers may also be used. Particularly useful polyether modified silicones include KF-6011, KF-6012, KF-6013, KF-6015, KF-6015, KF-6017, KF-6043, KF-6028, and KF-6038 from Shin-Etsu. Also particularly useful are the polyglycerolated linear or branched siloxane emulsifiers including KF-6100, KF-6104, and KF-6105 from Shin-Etsu. Emulsifiers also include emulsifying silicone elastomers. Suitable emulsifying silicone elastomers may include at least one polyalkyl ether or polyglycerolated unit.

As used herein, the term "cosmetically/dermatologically acceptable excipient" means any ingredient/compound or mixture of ingredients/compounds or compositions that are typically employed to produce other desirable effects in skin care/sunscreen compositions. The preferred cosmetically/dermatologically acceptable excipients include but not limited to preservatives, antioxidants, chelating agents, sunscreen agents, proteins, amino acids, vitamins, dyes, hair coloring agents, plant extracts, plant derivatives, plant tissue extracts, plant seed extracts, plant oils, botanicals, botanical extracts, humectants, fragrances, perfumes, oils, emollients, lubricants, butters, penetrants, thickeners, viscosity modifiers, thickeners, polymers, resins, hair fixatives, film formers, surfactants, detergents, emulsifiers, opacifying agents, volatiles, propellants, liquid vehicles, carriers, salts, pH adjusting agents, neutralizing agents, buffers, hair conditioning agents, anti-static agents, anti-frizz agents, anti-dandruff agents, hair waving agents, hair straightening agents, relaxers, absorbents, fatty substances, gelling agents, moisturizers, hydrophilic or lipophilic active agent, preserving agents, fillers, dyestuffs, reducing agents, cosmetic oils, perfumes, liquid vehicles, solvents, carriers, silicones, and combinations thereof.

Suitable emulsifiers include the following classes of ethers and esters: ethers of polyglycols and of fatty alcohols, esters of polyglycols and of fatty acids, ethers of polyglycols and of fatty alcohols which are glycosylated, esters of polyglycols and of fatty acids which are glycosylated, ethers of $C_{12-30}$ alcohols and of glycerol or of polyglycerol, esters of $C_{12-30}$ fatty acids and of glycerol or of polyglycerol, ethers of oxyalkylene-modified $C_{12-30}$ alcohols and of glycerol or polyglycerol, ethers of $C_{12-30}$ fatty alcohols comprising and of sucrose or of glucose, esters of sucrose and of $C_{12-30}$ fatty acids, esters of pentaerythritol and of $C_{12-30}$ fatty acids, esters of sorbitol and/or of sorbitan and of $C_{12-30}$ fatty acids, ethers of sorbitol and/or of sorbitan and of alkoxylated sorbitan, ethers of polyglycols and of cholesterol, esters of $C_{12-30}$ fatty acids and of alkoxylated ethers of sorbitol and/or sorbitan, and combinations thereof. Linear or branched type silicone emulsifiers may also be used.

The skin care and sunscreen composition of present application can be preserved by adding minor quantity of preservatives to the compositions. Such preservatives can be selected from, but are not limited to triazoles, imidazoles, naphthalene derivatives, benzimidazoles, morphline derivatives, dithiocarbamates, benzisothiazoles, benzamides, boron compounds, formaldehyde donors, isothiazolones, thiocyanates, quaternary ammonium compounds, iodine derivates, phenol derivatives, micobicides, pyridines, dialkylthiocarbamates, nitriles, parabens, alkyl parabens and salts thereof. The range of preservative employed is in the range of from about 0.01 wt. % to about 10 wt. %.

The preferred fatty substance based additive/excipient for the present application include fatty alcohols, natural and synthetic waxes, ceramides, mineral oils, vegetable oils, animal oils, synthetic oils. The other preferred fatty substance is isododecane, hydrogenated polyisobutene, squalane, isononyl isononanoate, cyclotetra- and - pentadimethicones, phenyltrimethicone, ethylene homopolymers, ethoxylated fats and oils, fluoroalkanes, seracite, shea butter, arachidyl propionate alone or in combination. For the definition of waxes, mention may be made, for example, of P. D. Dorgan, Drug and Cosmetic Industry, December 1983, pp. 30-33.

Moisturizers employed in the present application would include glycols, glycerols, propylene glycol, diethylene glycol monoethyl ether, sorbitol, sodium salt of pyroglutamic acid, glycerol, glycerol derivatives, glycerin, trehalose, sorbitol, maltitol, dipropylene glycol, 1,3-butylene glycol, sodium hyaluronate, and the like. Examples of humectants which can be incorporated into a product of the present application are glycerine, propylene glycol, polypropylene glycol, polyethylene glycol, lactic acid, sodium lactate, pyrrolidone carboxylic acid, urea, phospholipids, collagen, elastin, ceramides, lecithin sorbitol, PEG-4, and mixtures thereof. Additional suitable moisturizers are polymeric moisturizers that belong to water soluble and/or water swellable in nature. Polysaccharides such as hyaluronic acid, chitosan can also be employed along with moisturizers of the present application as binder to enhance their property. The range of moisturizer is from about 0.1 wt. % to about 10 wt. %.

The suitable solvent of the present application can consist of water, a cosmetically/dermatologically acceptable solvent, or a blend of water and a cosmetically/dermatologically acceptable solvent, such as a lower alcohol composed of $C_1$ to $C_4$, such as ethanol, isopropanol, t-butanol, n-butanol, alkylene glycols such as propylene glycol, and glycol ethers. The most preferred solvents of the present application would include water, ethanol and/or iso-propanol. It is contemplated to employ other suitable solvents for preparing products of the present application which would include but are not limited to, linear and branched $C_1$-$C_6$ alcohols, such as ethanol, propanol, isopropanol, butanol, hexanol, and mixtures thereof; aromatic alcohols, such as benzyl alcohol, cycloaliphatic alcohols, such as cyclohexanol, and the like; saturated $C_{12}$-$C_{30}$ fatty alcohol, such as lauryl alcohol, myristyl alcohol, cetyl alcohol, stearyl alcohol, behenyl alcohol, and the like. Non-limiting examples of polyols include polyhydroxy alcohols, such as glycerin, propylene glycol, butylene glycol, hexylene glycol, $C_2$-$C_4$ alkoxylated alcohols and $C_2$-$C_4$ alkoxylated polyols, such as ethoxylated, propoxylated, and butoxylated ethers of alcohols, diols, and polyols having about 2 to about 30 carbon atoms and 1 to about 40 alkoxy units, polypropylene glycol, polybutylene glycol, and the like. Non-limiting examples of nonaqueous auxiliary solvents include silicones, and silicone derivatives, such as cyclomethicone, and the like, aliphatic solvents such as cyclohexane and heptane, ketones such as acetone and methyl ethyl ketone, and mixtures thereof; ethers such as diethyl ether, dimethoxymethane, and mixtures thereof, natural and synthetic oils and waxes, such as vegetable oils, plant oils, animal oils, essential oils, mineral oils, $C_7$-$C_{40}$ isoparaffins, alkyl carboxylic esters, such as ethyl acetate, amyl acetate, ethyl lactate, and the like, jojoba oil, shark liver oil, and the like.

The alkaline pH adjusting agents such as alkali metal hydroxides, for example, sodium hydroxide, and potassium hydroxide; ammonium hydroxide; organic bases, such as triethanolamine, diisopropylamine, dodecylamine, diisopropanolamine, aminomethyl propanol, cocamine, oleamine, morpholine, triamylamine, triethylamine, tromethamine (2-amino-2-hydroxymethyl)-1,3-propanediol), and tetrakis(hydroxypropyl)ethylenediamine; and alkali metal salts of inorganic acids, such as sodium borate (borax), sodium phosphate, sodium pyrophosphate, and the like, and mixtures thereof. Acidic pH adjusting agents can be organic acids, including amino acids, and inorganic mineral acids. Non-limiting examples of acidic pH adjusting agents include acetic acid, citric acid, fumaric acid, glutamic acid, glycolic acid, hydrochloric acid, lactic acid, nitric acid, phosphoric acid, sodium bisulfate, sulfuric acid, tartaric acid, and the like, and mixtures thereof. The desired pH of the personal care composition is preferably between about 4 to about 8. The utility levels of the pH modifying agent can be present in an effective amount required to achieve the desired pH level.

The coloring agents, colorants or dyes used herein include natural foods colors and dyes suitable for food, drug and cosmetic applications. These colorants are also known as FD & C, and D&C dyes and lakes and are preferably water-soluble in nature. A full recitation of all FD&C and D&C dyes and their corresponding chemical structures may be found in the Kirk-Othmer Encyclopedia of Chemical Technology, Volume 5, pages 857-884, which text is accordingly incorporated herein by reference. These coloring agents may be incorporated in amount up to about 3%, more particularly up to about 2%, and in some cases less than about 1% by weight of the personal care compositions.

A perfume or fragrance obtained from natural or synthetic source can be employed in the present skin/sunscreen composition. The fragrance can be used along with a suitable solvent, diluents or carrier. Fragrances may be added in any conventionally known method, for example, admixing to a composition or blending with other ingredients used to form a composition, in amounts which are found to be useful to increase or impart the desired scent characteristics to the disinfectant or cleaning compositions. Fragrances for the present application can be one or more selected from the following non-limiting group of compounds such as essential oils, absolutes, resinoids, resins, concretes, hydrocarbons, alcohols, aldehydes, ketones, ethers, acids, esters, acetals, ketals, nitriles, including saturated and unsaturated compounds and aliphatic, carbocyclic and heterocyclic compounds.

The term "sequestering agent" or "chelating agent" as used herein relates to a compound which is capable of bonding or complexing a metal ion between two or more atoms of the compound, thereby neutralizing or controlling harmful effects of such metal ions. Wherein holding or bonding of a metal ion is through combination of one or more different types of bonds including coordination and/or ionic bonds. Further, the information on sequestering and chelating agents that are considered for the present application is duly disclosed in T. E. Furia, CRC Handbook of Food Additives, 2nd Edition, pp. 271-294 (1972), and M. S. Peterson and A. M. Johnson (Eds.), Encyclopedia of Food Science, pp. 694-699 (1978).

Non-limiting examples of suitable thickeners and/or viscosifiers include: Acetamide MEA; acrylamide/ethalkonium chloride acrylate copolymer; acrylamide/ethyltrimonium chloride acrylate/ethalkonium chloride acrylate copolymer; acrylamides copolymer; acrylamide/sodium acrylate copolymer; acrylamide/sodium acryloyldimethyltaurate copolymer; acrylates/acetoacetoxyethyl methacrylate copolymer; acrylates/beheneth-25 methacrylate copolymer; acrylates/$C_{10}$-$C_{30}$ alkyl acrylate crosspolymer; acrylates/ceteth-20 itaconate copolymer; acrylates/ceteth-20 methacrylate copolymer; acrylates/laureth-25 methacrylate copolymer; acrylates/palmeth-25 acrylate copolymer; acrylates/palmeth-25 itaconate copolymer; acrylates/steareth-50 acrylate copolymer; acrylates/steareth-20 itaconate copolymer; acrylates/steareth-20 methacrylate copolymer; acrylates/stearyl methacrylate copolymer; acrylates/vinyl isodecanoate crosspolymer; acrylic acid/acrylonitrogens copolymer; adipic acid/methyl DEA crosspolymer; agar; agarose; alcaligenes polysaccharides; algin; alginic acid; almondamide DEA; almondamidopropyl betaine; aluminum/magnesium hydroxide stearate; ammonium acrylates/acrylonitrogens copolymer; ammonium acrylates copolymer; ammonium acryloyldimethyltaurate/vinyl formamide copolymer; ammonium acryloyldimethyltaurate/VP copolymer; ammonium alginate; ammonium chloride; ammonium polyacryloyldimethyl taurate; ammonium sulfate; amylopectin; apricotamide DEA; apricotamidopropyl betaine; arachidyl alcohol; arachidyl glycol; arachis hypogaea (peanut) flour; ascorbyl methylsilanol pectinate; astragalus gummifer gum; attapulgite; avena sativa (oat) kernel flour; avocadamide DEA; avocadamidopropyl betaine; azelamide MEA; babassuamide DEA; babassuamide MEA; babassuamidopropyl betaine; behenamide DEA; behenamide MEA; behenamidopropyl betaine; behenyl betaine; bentonite; butoxy chitosan; caesalpinia spinosa gum; calcium alginate; calcium carboxymethyl cellulose; calcium carrageenan; calcium chloride; calcium potassium carbomer; calcium starch octenylsuccinate; C20-40 alkyl stearate; canolamidopropyl betaine; capramide DEA; capryl/capramidopropyl betaine; carbomer; carboxybutyl chitosan; carboxymethyl cellulose acetate butyrate; carboxymethyl chitin; carboxymethyl chitosan; carboxymethyl dextran; carboxymethyl hydroxyethylcellulose; carboxymethyl hydroxypropyl guar; carnitine; cellulose acetate propionate carboxylate; cellulose gum; ceratonia siliqua gum; cetearyl alcohol; cetyl alcohol; cetyl babassuate; cetyl betaine; cetyl glycol; cetyl hydroxyethylcellulose; chimyl alcohol; cholesterol/HDI/pullulan copolymer; cholesteryl hexyl dicarbamate pullulan; citrus aurantium dulcis (orange) peel extract; cocamide DEA; cocamide MEA; cocamide MIPA; cocamidoethyl betaine; cocamidopropyl betaine; cocamidopropyl hydroxysultaine; coco-betaine; cocohydroxysultaine; coconut alcohol; coco/oleamidopropyl betaine; coco-Sultaine; cocoyl sarcosinamide DEA; cornamide/cocamide DEA; cornamide DEA; croscarmellose; crosslinked bacillus/glucose/sodium glutamate ferment; cyamopsis tetragonoloba (guar) gum; decyl alcohol; decyl betaine; dehydroxanthan gum; dextrin; dibenzylidene sorbitol; diethanolaminooleamide DEA; diglycol/CHDM/isophthalates/SIP copolymer; dihydroabietyl behenate; dihydrogenated tallow benzylmonium hectorite; dihydroxyaluminum aminoacetate; dimethicone/PEG-10 crosspolymer; dimethicone/PEG-15 crosspolymer; dimethicone propyl PG-betaine; dimethylacrylamide/acrylic acid/polystyrene ethyl methacrylate copolymer; dimethylacrylamide/sodium acryloyldimethyltaurate crosspolymer; disteareth-100 IPDI; DMAPA acrylates/acrylic acid/acrylonitrogens copolymer; erucamidopropyl hydroxysultaine; ethylene/sodium acrylate copolymer; gelatin; gellan gum; glyceryl alginate; glycine soja (soybean) flour; guar hydroxypropyltrimonium chloride; hectorite; hyaluronic acid; hydrated silica; hydrogenated potato starch; hydrogenated tallow; hydrogenated tallowamide DEA; hydrogenated tallow betaine; hydroxybutyl methylcellulose; hydroxyethyl acrylate/sodium acryloyldimethyl taurate copolymer; hydroxyethylcellulose; hydroxyethyl chitosan; hydroxyethyl ethylcellulose; hydroxyethyl stearamide-MIPA; hydroxylauryl/hydroxymyristyl betaine; hydroxypropylcellulose; hydroxypropyl chitosan; hydroxypropyl ethylenediamine carbomer; hydroxypropyl guar; hydroxypropyl methylcellulose; hydroxypropyl methylcellulose stearoxy ether; hydroxypropyl starch; hydroxypropyl starch phosphate; hydroxypropyl xanthan gum; hydroxysteramide MEA; isobutylene/sodium maleate copolymer; isostearamide DEA; isostearamide MEA; isostearamide mIPA; isostearamidopropyl betaine; lactamide MEA; lanolinamide DEA; lauramide DEA; lauramide MEA; lauramide MIPA; lauramide/myristamide DEA; lauramidopropyl betaine; lauramidopropyl hydroxysultaine; laurimino bispropanediol; lauryl alcohol; lauryl betaine; lauryl hydroxysultaine; lauryl/myristyl glycol hydroxypropyl ether; lauryl sultaine; lecithinamide DEA; linoleamide DEA; linoleamide MEA; linoleamide MIPA; lithium magnesium silicate; lithium magnesium sodium silicate; macrocystis pyrifera (kelp); magnesium alginate; magnesium/aluminum/hydroxide/carbonate; magnesium aluminum silicate; magnesium silicate; magnesium trisilicate; methoxy PEG-22/dodecyl glycol copolymer; methylcellulose; methyl ethylcellulose; methyl hydroxyethylcellulose; microcrystalline cellulose; milkamidopropyl betaine; minkamide DEA; minkamidopropyl betaine;

MIPA-myristate; montmorillonite; Moroccan lava clay; myristamide DEA; myristamide MEA; myristamide MIPA; myristamidopropyl betaine; myristamidopropyl hydroxysultaine; myristyl alcohol; myristyl betaine; natto gum; nonoxynyl hydroxyethylcellulose; oatamide MEA; oatamidopropyl betaine; octacosanyl glycol isostearate; octadecene/MA copolymer; oleamide DEA; oleamide MEA; oleamide MIPA; oleamidopropyl betaine; oleamidopropyl hydroxysultaine; oleyl betaine; olivamide DEA; olivamidopropyl betaine; oliveamide MEA; palmamide DEA; palmamide MEA; palmamide MIPA; palmamidopropyl betaine; palmitamide DEA; palmitamide MEA; palmitamidopropyl betaine; palm kernel alcohol; palm kernelamide DEA; palm kernelamide MEA; palm kernelamide MIPA; palm kernelamidopropyl betaine; peanutamide MEA; peanutamide MIPA; pectin; PEG-800; PEG-crosspolymer; PEG-150/decyl alcohol/SMDI copolymer; PEG-175 diisostearate; PEG-190 distearate; PEG-15 glyceryl tristearate; PEG-140 glyceryl tristearate; PEG-240/HDI copolymer bis-decyltetradeceth-20 ether; PEG-100/IPDI copolymer; PEG-180/laureth-50/™MG copolymer; PEG-10/lauryl dimethicone crosspolymer; PEG-15/lauryl dimethicone crosspolymer; PEG-2M; PEG-5M; PEG-7M; PEG-9M; PEG-14M; PEG-20M; PEG-23M; PEG-25M; PEG-45M; PEG-65M; PEG-90M; PEG-115M; PEG-160M; PEG-180M; PEG-120 methyl glucose trioleate; PEG-180/octoxynol-40/™MG copolymer; PEG-150 pentaerythrityl tetrastearate; PEG-4 rapeseedamide; PEG-150/stearyl alcohol/SMDI copolymer; phaseolus angularis seed powder; polianthes tuberosa extract; polyacrylate-3; polyacrylic acid; polycyclopentadiene; polyether-1; polyethylene/isopropyl maleate/MA copolyol; polyglyceryl-3 disiloxane dimethicone; polyglyceryl-3 polydimethylsiloxyethyl dimethicone; polymethacrylic acid; polyquaternium-52; polyvinyl alcohol; potassium alginate; potassium aluminum polyacrylate; potassium carbomer; potassium carrageenan; potassium chloride; potassium palmate; potassium polyacrylate; potassium sulfate; potato starch modified; PPG-2 cocamide; PPG-1 hydroxyethyl caprylamide; PPG-2 hydroxyethyl cocamide; PPG-2 hydroxyethyl coco/isostearamide; PPG-3 hydroxyethyl soyamide; PPG-14 laureth-60 hexyl dicarbamate; PPG-14 laureth-60 isophoryl dicarbamate; PPG-14 palmeth-60 hexyl dicarbamate; propylene glycol alginate; PVP/decene copolymer; PVP montmorillonite; pyrus cydonia seed; pyrus malus (apple) fiber; rhizobian gum; ricebranamide DEA; ricinoleamide DEA; ricinoleamide MEA; ricinoleamide MIPA; ricinoleamidopropyl betaine; ricinoleic acid/adipic acid/AEEA copolymer; rosa multiflora flower wax; sclerotium gum; sesamide DEA; sesamidopropyl betaine; sodium acrylate/acryloyldimethyl taurate copolymer; sodium acrylates/acrolein copolymer; sodium acrylates/acrylonitrogens copolymer; sodium acrylates copolymer; sodium acrylates crosspolymer; sodium acrylate/sodium acrylamidomethylpropane sulfonate copolymer; sodium acrylates/vinyl isodecanoate crosspolymer; sodium acrylate/vinyl alcohol copolymer; sodium carbomer; sodium carboxymethyl chitin; sodium carboxymethyl dextran; sodium carboxymethyl beta-glucan; sodium carboxymethyl starch; sodium carrageenan; sodium cellulose sulfate; sodium chloride; sodium cyclodextrin sulfate; sodium hydroxypropyl starch phosphate; sodium isooctylene/MA copolymer; sodium magnesium fluorosilicate; sodium oleate; sodium palmitate; sodium palm kernelate; sodium polyacrylate; sodium polyacrylate starch; sodium polyacryloyldimethyl taurate; sodium polygammaglutamate; sodium polymethacrylate; sodium polystyrene sulfonate; sodium silicoaluminate; sodium starch octenylsuccinate; sodium stearate; sodium stearoxy PG-hydroxyethylcellulose sulfonate; sodium styrene/acrylates copolymer; sodium sulfate; sodium tallowate; sodium tauride acrylates/acrylic acid/acrylonitrogens copolymer; sodium tocopheryl phosphate; solanum tuberosum (potato) starch; soyamide DEA; soyamidopropyl betaine; starch/acrylates/acrylamide copolymer; starch hydroxypropyltrimonium chloride; stearamide AMP; stearamide DEA; stearamide DEA-distearate; stearamide DIBA-stearate; stearamide MEA; stearamide MEA-stearate; stearamide MIPA; stearamidopropyl betaine; steareth-60 cetyl ether; steareth-100/PEG-136/HDI copolymer; stearyl alcohol; stearyl betaine; sterculia urens gum; synthetic fluorphlogopite; tallamide DEA; tallow alcohol; tallowamide DEA; tallowamide MEA; tallowamidopropyl betaine; tallowamidopropyl hydroxysultaine; tallowamine oxide; tallow betaine; tallow dihydroxyethyl betaine; tamarindus indica seed gum; tapioca starch; TEA-alginate; TEA-carbomer; TEA-hydrochloride; trideceth-2 carboxamide MEA; tridecyl alcohol; triethylene glycol dibenzoate; trimethyl pentanol hydroxyethyl ether; triticum vulgare (wheat) germ powder; triticum vulgare (wheat) kernel flour; triticum vulgare (wheat) starch; tromethamine acrylates/acrylonitrogens copolymer; tromethamine magnesium aluminum silicate; undecyl alcohol; undecylenamide DEA; undecylenamide MEA; undecylenamidopropyl betaine; welan gum; wheat germamide DEA; wheat germamidopropyl betaine; xanthan gum; yeast beta-glucan; yeast polysaccharides; zea mays (corn) starch; and blends thereof.

Yet another embodiment of the present application discloses that the pH of the composition is in the range of from about 2 to about 12. Other possible ranges of pH can be about 2 to about 4, about 4 to about 6, about 6 to about 8, about 8 to about 10, about 10 to about 12.

According to one embodiment of the present application, the skin care and sun screen compositions of the present application also impart a dry and powdery feel and decreased shine appearance on to surface of skin of humans after their application.

Further, certain aspects of the present invention are illustrated in detail by way of the following examples. The examples are given herein for illustration of the invention and are not intended to be limiting thereof.

**EXAMPLES**

**Example 1a, 1b & 1c:** Non-ionic Based

The process for preparing skin care/sunscreen (non-ionic based) is described in example-1(a). Various ingredients including their weight proportions used for preparing the skin care composition are disclosed **Table-1.** In a first step, Phase A was prepared, wherein, 2.00 % w/w of Propylene glycol was mixed with 56.78 % w/w water in a beaker under continuous stirring at a temperature of 75°C to obtain a homogenized mixture. Thereafter, 0.30 % w/w Carbopol was added under continuous stirring until a smooth and uniform mixture free with lumps was obtained. 0.30 % w/w methyl paraben preservative was then added to the mixture to obtain a resultant Phase A solution. Simultaneously, Phase B components corresponding to example-1a and listed in Table-1 were mixed together under continuous stirring at a temperature of 75°C to obtain a yellow colored clear solution of phase B. This Phase B solution was then added to the Phase A solution under continuous stirring to obtain a mixture of Phase A and Phase B which is off white in color. The mixture of Phase A and Phase B was slowly cooled to 65°C and subsequently mixed with Phase C components corresponding to example-1a listed in Table-1. The pH of the mixture of Phase A, Phase B and Phase C was maintained at around 5.6. Thereafter, 1.0 % w/w of vinyl pyrrolidone/acrylic acid/lauryl methacrylate copolymer was added to the mixture of Phase A, Phase B and Phase C to obtain a resultant skin care/sunscreen composition. The pH of resultant composition was maintained at 5.5-6.0.

Another set of skin care/sunscreen compositions (corresponding to Examples 1b & 1c) are also prepared in the same manner as for Example 1a. In Example 1b, a copolymer of vinyl pyrrolidone and long chain alpha-olefins (Ganex V220) were used in-place of vinyl pyrrolidone/acrylic acid/lauryl methacrylate copolymer, whereas in Example 1c, no polymer was used.

**Table-1:** Non-ionic base compositions

| Phase | Ingredients | Example-1a % w/w | Example-1b % w/w | Example-1c % w/w |
|---|---|---|---|---|
| A | Deisonized Water | 56.78 | 57.82 | 57.82 |
| | Propylene glycol | 2.00 | 2.0 | 2.0 |
| | Carbomer | 0.30 | 0.30 | 0.30 |
| | Methyl paraben | 0.30 | 0.30 | 0.30 |
| B | Homosalate | 10.00 | 10.00 | 10.00 |
| | Ethylhexyl salicylate (Octisalate) | 5.00 | 5.00 | 5.00 |
| | Octocrylene | 5.00 | 5.00 | 5.00 |
| | VP/Eicosene Copolymer | 0.00 | 1.000 | 0.000 |
| | Benzophenone-3 | 6.00 | 6.00 | 6.00 |
| | Butyl Methoxydibenzoylmethane | 3.00 | 3.00 | 3.00 |
| | Diisopropyl Adipate | 2.00 | 2.00 | 2.00 |
| | Glyceryl Stearate (and) PEG-100 stearate | 4.00 | 4.00 | 4.00 |
| C | NaOH (Pellets) | 0.08 | 0.08 | 0.08 |
| | water | 3.00 | 3.00 | 3.00 |
| | pH | 5.5-6.0 | 5.5-6.0 | 5.5-6.0 |
| D | VP/Acrylates/Lauryl Methacrylate Copolymer | 1.00 | 0.00 | 0.00 |
| | Phenoxyethanol (and) Caprylyl Glycol (and) Sorbic Acid | 1.50 | 1.50 | 1.50 |
| | Initial Contact Angle (°) | 42.83 | - | 49.71 |
| | Contact angle after immersion in water (°) | 68.15 | - | 51.70 |

**Water resistance test method:** VITRO-SKIN® (VS) is cut into 3 X 4 *cm* strips and is mounted in place in a 35mm slide holder, and four slides are made for each test product and wherein, one slide is made for the blank to obtain the baseline and zero readings. The slides are then placed into a Thermotron hydration chamber for 16-18 hours to allow the VS to hydrate, wherein, the relative humidity of the chamber is maintained at 45% and the temperature at 30°C and 7mg of the test product is applied to each strip of VS. The slide is then placed back in the humidity chamber for 20 minutes to allow for coalescence of the test product. The initial UV absorbance of each slide is measured with a UV-Vis spectrophotometer in the wavelength range of 290-400nm. Two readings are taken for each slide - one from each end. The slides are then immersed in a 30°C water bath, with circulation of 90*rpm* for 80 minutes. The slides are then removed and excess water shaken off. The slides are equilibrated in the humidity chamber for 15 minutes, and the absorbance post immersion is calculated. Area under the curve (*sum of the y value*), from 290-400nm is calculated for each reading. This is done for both initial readings and post immersion readings. The percent water resistance is calculated from the following equation:

$$\text{Percent Water Resistance} = (\text{Absorbance post immersion} / \text{Initial Absorbance}) \times 100.$$

The average of the 4 slides give the final percent water resistance.

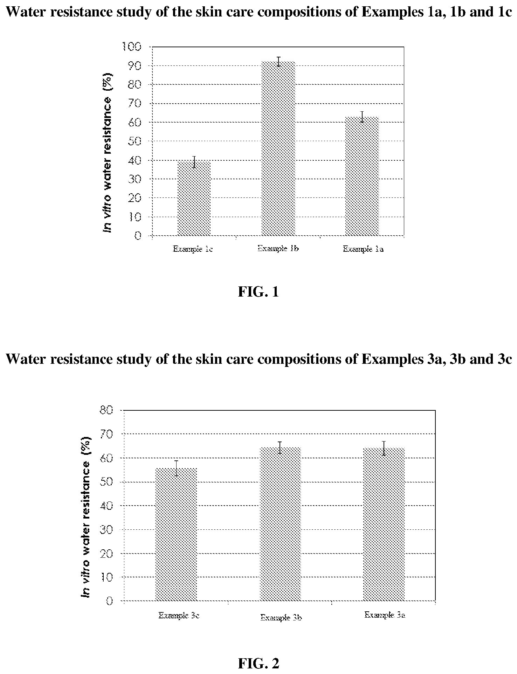

Water resistance property of the skin care compositions of examples 1 a, 1b and 1c were evaluated and presented in **Figure 1** of the accompanying drawings. Further, the contact angle of the composition of example-1a is also measured and compared with the contact angle of the composition of example-1c (without the terpolymer). As evident from the data of table-1, there is an increase of 25.32° in the contact angle of the composition of example-1a, whereas an increase of only 1.99° in observed in the contact angle of the composition of example-1c which comprises no terpolymer. It is observed that the contact angle of the skincare composition exhibited an increase in contact angle of at least 5° after immersion in water, as compared to the composition devoid of the terpolymer of the present application. The contact angle of skincare composition of the present application was measured in accordance with the method disclosed in US20130243703.

**Example-2a, 2b & 2c:** Non-ionic base compositions having phenoxyethanol and alkyl parabens as preservative

The process for preparing the skin care/sunscreen composition (non-ionic base compositions) is described in Example-2(a). Various ingredients including their weight proportions used for preparing the skin care/sun screen composition of Example-2 are disclosed Table-2. In a first step, 2.0 % w/w Propylene glycol and 53.73 % w/w water were mixed together in a beaker under continuous stirring at a temperature of 75°C to obtain a homogenized mixture. Thereafter, 0.20 % w/w of Carbopol was sprinkled under continuous stirring until a smooth and uniform mixture free with lumps was obtained. Thereafter, 1.0 % w/w LiquaPar MEP preservative (Phenoxyethanol and Methylparaben and Ethylparaben and Caprylyl Glycol) was added under continuous stirring to obtain a Phase A solution. Simultaneously, the Phase B components corresponding to example 2a listed in Table-2 were mixed together under continuous stirring at a temperature of 75°C to obtain a yellow colored and clear solution of Phase B. The Phase B solution was then added to the Phase A solution under continuous stirring to obtain a mixture of Phase A and Phase B. The obtained mixture of Phase A and Phase B was then slowly cooled to 65°C and mixed with Phase C components corresponding to example 2a listed in Table-2 to obtain a mixture of Phase A, Phase B, and Phase C. 1.0 % w/w of vinyl pyrrolidone/acrylic acid/lauryl methacrylate was then added to the mixture of Phase A, Phase B and Phase C under continuous stirring at a temperature of 55°C to obtain the resultant composition. pH of the composition was adjusted around 5.24 with phase E components listed in **Table-2.**

Another set of skin care compositions (corresponding to Examples 2b & 2c) were also prepared in the same manner as for Example 2a. In Example 2b, a copolymer of vinyl pyrrolidone and long chain alpha-olefins (Ganex V220; VP/Eicosene Copolymer having relative viscosity of 1.364) was used instead of the copolymer of vinyl pyrrolidone/acrylic acid/lauryl methacrylate; In Example 2c, no polymer was used.

**Table-2:** Non-ionic base compositions having phenoxyethanol and alkyl parabens as preservative

| Phase | Ingredients | Example-2a % w/w | Example-2b % w/w | Example-2c % w/w |
|---|---|---|---|---|
| A | Water | 53.73 | 58.64 | 58.64 |
| | Propylene glycol | 2.00 | 2.0 | 2.0 |
| | Phenoxyethanol (and) Methyl paraben (and) ethylparaben (and) propylparaben | 1.0 | 1.0 | 1.0 |
| | Carbomer | 0.20 | 0.30 | 0.30 |
| B | Homosalate | 10.00 | 10.00 | 10.00 |
| | Ethylhexyl salicylate (Octisalate) | 5.00 | 5.00 | 5.00 |
| | Octocrylene | 5.00 | 5.00 | 5.00 |
| | Benzophenone-3(oxybenzone) | 6.00 | 6.00 | 6.00 |

(continued)

| Phase | Ingredients | Example-2a % w/w | Example-2b % w/w | Example-2c % w/w |
|---|---|---|---|---|
| | Butyl Methoxydibenzoylmethane | 3.00 | 3.00 | 3.00 |
| | Diisopropyl Adipate | 2.00 | 2.00 | 2.00 |
| | **VP/Eicosene Copolymer** | 0.00 | **1.000** | 0.000 |
| | Cetyl alcohol | 2.00 | 0.00 | 0.00 |
| | Glyceryl Stearate (and) PEG-100 stearate | 4.00 | 4.00 | 4.00 |
| C | NaOH (Pellets) | 0.07 | 0.06 | 0.06 |
| | Water | 5.00 | 3.00 | 3.00 |
| D | **VP/Acrylates/Lauryl Methacrylate Copolymer** | **1.00** | 0.00 | 0.00 |

Water resistance properties of the skin care compositions of Examples 2 a, 2b and 2c were duly evaluated.

**Example-3a & 3b 7 3c:** Lamellar gel base

Example 3a describes a process for preparing skin care composition in lamellar gel base form. Various ingredients including their weight proportions were used for preparing the skin care/sun screen composition of example-3 are disclosed Table-3. In a first step, 60.99 % w/w of water, 0.05 % w/w of disodium EDTA, 3.0 % w/w of Lubrajel, 1.0 % w/w of Phenoxyethanol and Caprylyl Glycol, and 0.30 % w/w of methylparaben were mixed together in a step wise manner under continuous stirring to obtain a homogeneous mixture. 0.30 % w/w of carbomer was then sprinkled to the mixture under continuous stirring at a temperature of 75°C to obtain a smooth and homogeneous mixture of Phase A and Phase B. In a separate beaker, all the Phase C components corresponding to the Example 3a listed in Table-3 were mixed together and heated at a temperature of 80°C to obtain a yellow colored clear solution. The temperature of the Phase C solution was then maintained at 75°C and then gradually added to the mixture of Phase A and Phase B under continuous stirring to obtain a mixture of Phase A, Phase B, and Phase C. In a separate beaker, phase D components corresponding to example-3a listed in table-3 were mixed together at room temperature. The phase D solution was then added to the mixture of Phases A, B and C at 50°C. 1.0 % w/w Phase E (Vinylpyrrolidone/acrylic acid/lauryl methacrylate copolymer) was added to the mixture of Phase A, Phase B, Phase C and Phase D at 55°C to obtain a resultant composition. The pH of the resultant composition was maintained at 6. The resultant composition was then cooled to 25°C.

Another set of skin care compositions (corresponding to Examples 3 b & 3 c) were also prepared in the same manner as for Example 3a. In Example 3b, a copolymer of vinyl pyrrolidone and long chain alpha-olefins (Ganex V220; VP/Eicosene copolymer having relative viscosity of 1.364) was used instead of the copolymer of vinyl pyrrolidone/acrylic acid/lauryl methacrylate; In Example 3c, no polymer was used.

**Table-3:** Lamellar gel base composition

| Phase | Ingredients | Example-3a % w/w | Example-3b % w/w | Example-3c % w/w |
|---|---|---|---|---|
| A | Water | 60.99 | 61.02 | 62.02 |
| | Disodium EDTA | 0.05 | 0.05 | 0.05 |
| | Water (and) Glycerin (and) glyceryl Acrylate/Acrylic Acid Copolymer (and) PVM/MA copolymer | 3.0 | 3.0 | 3.0 |
| | Phenoxyethanol (and) Caprylyl Glycol | 1.0 | 1.0 | 1.0 |
| | Methylparaben | 0.30 | 0.30 | 0.30 |
| B | Carbomer | 0.30 | 0.30 | 0.30 |
| C | Glyceryl Stearate (and) Behenyl Alcohol (and) Palmitic Acid (and) Stearic Acid (and) Lecithin (and) Lauryl Alcohol (and) Myristyl Alcohol (and) Cetyl Alcohol | 4.00 | 4.00 | 4.00 |
| | Ceteareth-20 | 0.10 | 0.10 | 0.10 |
| | **VP/Eicosene Copolymer** | **0.00** | **1.00** | **0.00** |
| | Butyl Methoxydibenzoylmethane | 3.00 | 3.00 | 3.00 |

(continued)

| Phase | Ingredients | Example-3a % w/w | Example-3b % w/w | Example-3c % w/w |
|---|---|---|---|---|
| | Ethylhexyl Salicylate | 5.00 | 5.00 | 5.00 |
| | Octocrylene | 8.00 | 8.00 | 8.00 |
| | Homosalate | 10.00 | 10.00 | 10.00 |
| | Dimethicone | 2.00 | 2.00 | 2.00 |
| | Propylparaben | 0.15 | 0.15 | 0.15 |
| D | Sodium Hydroxide | 0.11 | 0.08 | 0.08 |
| | Water | 1.00 | 1.00 | 1.00 |
| E | VP/acrylate/lauryl methacrylate copolymer | **1.00** | 0.00 | 0.00 |

Water resistance properties of the skin care compositions of Examples 3a, 3b and 3c were evaluated and presented in **Figure 2** of the accompanying drawings.

**Examples-4a & 4b:** Anionic Base

Example 4a describes a process for preparing skin care/sunscreen (anionic base compositions). In a first process step, Phase A was prepared. For this 45.70 % w/w water was added to a beaker and heated. Rest of the Phase A components corresponding to Example 4a and listed in Table-4 were then added to the water under continuous stirring to obtain Phase A solution. Subsequently, 0.50 % w/w of acrylic acid/VP crosspolymer was added to the Phase A under continuous stirring at 75°C to obtain a homogeneous mixture of Phase A and Phase B. In a separate beaker, all the Phase C components corresponding to Example 4a in Table-4, were mixed together and heated at 75°C to obtain a yellow colored clear solution of Phase C. The Phase C solution was then added to the homogeneous mixture of Phase A & Phase B under continuous stirring. The temperature of the mixture of phases A, B and C is lowered to 65°C. Simultaneously, in a separate beaker, Phase D components corresponding to Example-4a in **Table-4** were mixed together and added to the mixture of phases A, B and C at 65°C. 1.0 % w/w of Phase E (Vinylpyrrolidone/acrylic acid/lauryl methacrylate copolymer) was added under continuous stirring at 55°C to obtain a resultant composition. The pH of the resultant composition was adjusted to 6, if necessary. The resultant composition was cooled to 25°C.

Another set of skin care composition (corresponding to Example 4b) was also prepared in the same manner as for Example 4a. In Example 4b, no polymer was used

**Table-4:** Anionic base emulsion composition

| Phase | Ingredients | Example-4a % w/w | Example-4b % w/w |
|---|---|---|---|
| A | DI Water | 45.70 | 47.50 |
| | Glycerin | 5.00 | 5.00 |
| | Propylene Glycol | 5.00 | 5.00 |
| | Disodium EDTA | 0.10 | 0.10 |
| | Methylparaben NF | 0.3 | 0.3 |
| | Sodium Hydroxide (10% aq. soln) | 0.30 | 0.30 |
| B | Acrylic Acid/VP Crosspolymer | 0.50 | 0.50 |
| C | Cetearyl alcohol, Dicetyl Phosphate, and Ceteth-10 Phosphate) | 3.50 | 3.50 |
| | Ceteareth-20 | 0.60 | 0.60 |
| | Diisopropyl adipate | 4.00 | 4.00 |
| | Butyl Methoxydibenzoylmethane | 3.00 | 3.00 |
| | Benzophenone-3 | 6.00 | 6.00 |
| | Octocrylene | 5.00 | 5.00 |
| | Ethylhexyl Salicylate | 5.00 | 5.00 |
| | Homosalate | 10.00 | 10.00 |
| D | DI water | 2.00 | 2.00 |
| | Sodium Hydroxide 910 % aq soln) | 1.50 | 0.70 |

(continued)

| Phase | Ingredients | Example-4a % w/w | Example-4b % w/w |
|---|---|---|---|
| E | **VP/acrylate/lauryl methacrylate copolymer** | **1.00** | **0.00** |
| | Phenoxyethanol (and) Caprylyl Glycol (and) Sorbic acid) | 1.50 | 1.50 |

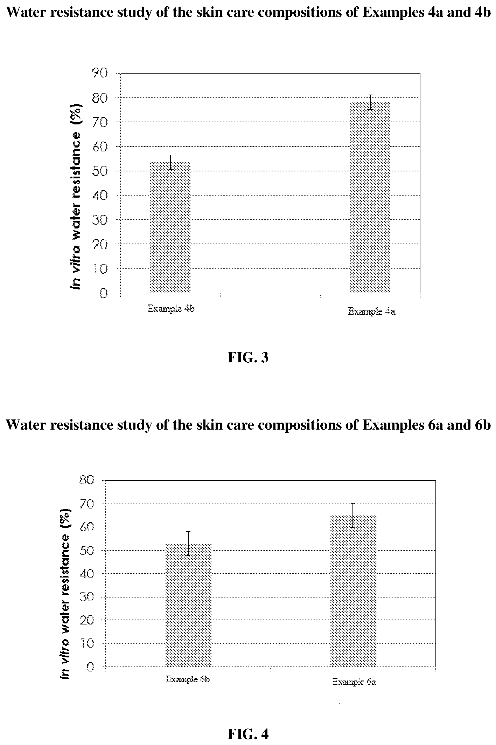

Water resistance properties of the skin care compositions of examples 4a and 4b were evaluated and presented in **Figure 3** of the accompanying drawings.

**Example-5a, 5b & 5c:** Non-ionic emulsion

Example 5a describes a process for preparing non-ionic base emulsion compositions. In a first process step, 76.80 % w/w water, 0.050 % w/w Tetrasodium EDTA, and 3.0 % w/w Lubrajel were added together in a beaker and heated at 75°C while heating, 0.60 % w/w acrylic acid/VP cross-polymer was also added to the beaker to obtain a homogeneous mixture of phase A & phase B. In a separate beaker, all Phase C components corresponding to example 5a listed in Table-5 were mixed together under continuous stirring until watery white and hazy solution is obtained. The phase C solution is then added to the homogeneous mixture of phase A & phase B under continuous stirring. In a separate beaker, all phase D components corresponding to Example-5a in Table-5 were mixed together under continuous stirring at 75°C. The phase D solution was then added to the main beaker having a mixture of Phase A, B and C to obtain a mixture of Phase A, Phase B, Phase C, and Phase D. In a separate process step, phase E components corresponding to Example 5a in Table 5 were added to obtain a clear and watery white solution of Phase E. The phase E solution was then added to the mixture of Phases A, B, C and D at 55°C and mixed well. Followed to this, 1.0 % w/w of Phase F (Vinylpyrrolidone/acrylic acid/lauryl methacrylate copolymer) was added to the mixture of phases A, B, C, D and E under continuous stirring at 55°C to obtain a resultant composition. The pH of the resultant composition was adjusted to 6, if necessary. The resultant composition was cooled to 25°C.

Another set of skin care compositions (corresponding to Examples 5 b & 5 c) are also prepared in the same manner as for Example 5a. In example 5b, Aluminum starch Octenylsuccinate was used instead of the copolymer of vinyl pyrrolidone/acrylic acid/lauryl methacrylate; In Example 5c, no polymers are used.

**Table-5:** Non-ionic emulsion compositions

| Phase | Ingredients | Example-5a % w/w | Example-5b % w/w | Example-5c % w/w |
|---|---|---|---|---|
| A | DI Water | 76.80 | 76.80 | 77.80 |
| | Tetrasodium EDTA | 0.050 | 0.050 | 0.05 |
| | Glycerin (and) glyceryl Acrylate/Acrylic Acid (and) propylene glycol polymer | 3.00 | 3.00 | 3.0 |
| | Phenoxyethanol & methylparaben & ethylparaben & propylparaben | 1.00 | 1.00 | 1.0 |
| B | Acrylic acid/VP cross-polymer | 0.60 | 0.60 | 0.60 |
| C | NaOH Pearls DI Water | 0.02 0.50 | 0.02 0.50 | 0.02 0.50 |
| D | PEG-100 Stearate & Glyceryl Stearate | 2.00 | 2.00 | 2.00 |
| | Butyrospermum Parkii (Shea Butter) | 2.00 | 2.00 | 2.00 |
| | Cetyl Lactate (Ceraphyl 28) | 2.00 | 2.00 | 2.00 |
| | Isocetyl Stearoyl Stearate | 2.50 | 2.50 | 2.50 |
| | Octyldodecyl Stearate | 3.50 | 3.50 | 3.50 |
| | Ethylhexyl Palmitate | 4.50 | 4.50 | 4.50 |
| E | Sodium Hydroxide | 0.03 | 0.03 | 0.03 |
| | Water | 0.50 | 0.50 | 0.50 |
| F | **VP/Acrylates/lauryl methacryate** | **1.00** | **0.00** | **0.00** |

(continued)

| Phase | Ingredients | Example-5a % w/w | Example-5b % w/w | Example-5c % w/w |
|---|---|---|---|---|
| | Aluminum Starch Octenylsuccinate | 0.00 | 1.00 | 0.00 |

**Example-6:** SUNSory active Fluid SPF 50 plus

Table-6: SUNSory active Fluid SPF 50 plus composition

| Phase | Ingredients | Example 6a % w/w | Example 6b % w/w |
|---|---|---|---|
| Phase A | Water/Aqua | 53.87 | 54.62 |
| | Tetrasodium EDTA | 0.1 | 0.1 |
| | Water/Aqua (and) Glycerin (and) Glyceryl Acrylate/Acrylic Acid Copolymer (and) PVM/MA Copolymer | 5 | 5 |
| Phase B | Acrylic Acid/VP Crosspolymer | 0.3 | 0.3 |
| Phase C | Potassium Cetyl Phosphate | 1.5 | 1.5 |
| Phase D | Diisopropyl Adipate | 2 | 2 |
| | Behenyl Alcohol (and) Decaglyceryl Pentasteareate (and) Sodium Stearoyl Lactate | 1.5 | 1.5 |
| | Bis-Ethylhexyloxyphenol Methoxyphenyl Triazine | 4 | 4 |
| | Butyl Methoxydibenzoylmethane | 4 | 4 |
| | Ethylhexyl Salicylate | 5 | 5 |
| | Octocrylene | 5 | 5 |
| | Homosalate | 8 | 8 |
| | Ethylhexyl triazone | 5 | 5 |
| | Dimethicone | 2 | 2 |
| | **VP/acrylates/lauryl methacrylate copolymer** | **0.7** | **0.0** |
| Phase E | Methylparaben | 0.3 | 0.3 |
| | Phenoxyethanol (and) Benzoic Acid (and) Dehydroacetic Acid | 1 | 1 |
| Phase F | Water/Aqua | 0.5 | 0.5 |
| | Sodium Hydroxide | 0.08 | 0.03 |
| Phase G | Parfum/Fragrance | 0.15 | 0.15 |

In-vitro water resistance study of the SUNsory Active Fluid SPF 50 plus is illustrated in **Figure 4** of the accompanying drawings.

**Example-7:** SUNsory light daily cream-in-gel SPF 20 Low emulsifier

Table-7: SUNsory light daily cream-in-gel SPF 20 Low emulsifier

| Phase | Ingredients | Example 7a % w/w | Example 7b % w/w |
|---|---|---|---|
| Phase A | Water/aqua | 55.88 | 57.01 |
| | Glycerin (and) Glyceryl Acrylate/Acrylic Acid Copolymer (and) Butylene Glycol (and) PVM/MA Copolymer | 5 | 5 |
| | Phenoxyethanol (and) Benzoic Acid (and) Dehydroacetic Acid | 0.9 | 0.9 |
| | Methylparaben | 0.2 | 0.2 |

(continued)

| Phase | Ingredients | Example 7a % w/w | Example 7b % w/w |
|---|---|---|---|
| | Tetrasodium EDTA | 0.1 | 0.1 |
| Phase B | Acrylic Acid/VP Crosspolymer | 0.3 | 0.3 |
| Phase C | Sodium Polyacrylate (and) Hydrogenated Polydecene (and) Trideceth-6 | 0.6 | 0.6 |
| Phase D | Glyceryl Stearate Citrate (and) Polyglyceryl-3 stearate (and) Hydrogenated lecithin | 0.3 | 0.3 |
| | Butyl Methoxydibenzoylmethane (Avobenzone) | 3 | 3 |
| | Bis-Ethylhexyloxyphenol Methoxyphenyl Triazine | 3 | 3 |
| | Ethylhexyl Salicylate | 5 | 5 |
| | Octocrylene | 4 | 4 |
| | Homosalate | 5 | 5 |
| | C12-15 Alkyl Lactate | 5 | 5 |
| | Dimethicone | 5 | 5 |
| | VP/acrylates/lauryl methacrylate copolymer | 1 | / |
| Phase E | Alcohol | 5 | 5 |
| Phase F | Sodium Hydroxide | 0.22 | 0.09 |
| | Water/Aqua | 0.5 | 0.5 |

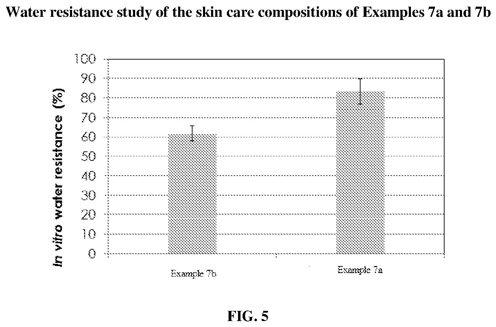

In-vitro water resistance study of the SUNsory light daily cream-in-gel SPF 20 with low emulsifier is illustrated in **Figure 5** of the accompanying drawings.

**Example 8:** SUNsory rebound balm-in-gel SPF 20 without emulsifier

**Table-8:** SUNsory rebound balm-in-gel SPF 20 without emulsifier

| Phase | Ingredients | Example 8 % w/w |
|---|---|---|
| Phase A | Water/aqua | 63.55 |
| | Tetrasodium EDTA | 0.05 |
| | Water/aqua (and) Glycerin (and) Glyceryl Acrylate/Acrylic Acid | 3 |
| | Copolymer (and) PVM/MA Copolymer PVP | 1.5 |
| | Phenoxyethanol & Methylparaben & Ethylparaben & Propylparaben | 0.7 |
| | Phenoxyethanol (and) Caprylyl Glycol | 0.5 |
| Phase B | Butyl Methoxydibenzoylmethane (Avobenzone) | 3 |
| | Octocrylene | 7 |
| | Ethylhexyl Salicylate | 5 |
| | Homosalate | 10 |
| | Dimethicone | 3.5 |
| | VP/acrylates/lauryl methacrylate copolymer | 0.8 |
| Phase C | Sodium Polyacrylate | 0.8 |
| Phase D | Sodium Hydroxide | 0.1 |
| | Water/Aqua | 0.5 |

**Example 9:**

This example describes a process for preparing the skin care/sunscreen composition based on a mixture comprising the aqueous as the discontinuous phase and the silicone phases as the continuous phase. Various ingredients including their weight proportions used for preparing the skin care composition are disclosed **Table-9.** All the phases *i.e.*

Phase A, Phase B and Phase C as listed in Table-9 were prepared in a separate step. for preparing the Phase A, 38% w/w of water was added to a beaker. Followed to this 0.50 % w/w of magnesium sulfate together with glycerin and Propylene glycol (and) Diazolidinyl Urea (and) Methylparaben (and) Propylparaben was added to the beaker under continuous stirring. In a separate step, Phase B was prepared wherein all the ingredients corresponding to Phase B as listed in Table-9 are mixed with heating at 60 to 65°C under continuous stirring until a uniform solution was obtained. Both the phases i.e. Phase A and Phase B were slowly added together under continuous stirring until a uniform mixture was obtained. In a separate step, Phase C was also prepared by mixing the components in weight proportion as listed in Table-9. The phase c was then slowly added to the uniform mixture of Phase A and Phase to obtain the composition.

**Table-9:** Aqueous/silicone base formula

| Phase | Ingredients | Example-9 % w/w |
|---|---|---|
| A | Water | 38.00 |
| | Sodium hydroxide (10% aq. Soln) | 1.80 |
| | Sodium Chloride | 0.00 |
| | Magnesium Sulfate | 0.50 |
| | Glycerin | 6.00 |
| | Propylene glycol (and) Diazolidinyl Urea (and) Methylparaben (and) Propylparaben | 0.50 |
| B | Dimethicone (and) Dimethicone/PEG-10/15 Crosspolymer | 1.00 |
| | Lauryl PEG-9 Polymethylsiloxyethyl Dimethicone | 2.50 |
| | Ethylhexyl Palmitate | 7.00 |
| | Isostearyl Neopentanoate | 7.50 |
| | Dimethicone | 6.00 |
| | Titanium Dioxide | 8.00 |
| C | NaOH (10% aq. Soln.) | 0.20 |
| | water | 20.00 |
| | VP/Acrylates/Lauryl Methacrylate Copolymer | 1.00 |
| | Total | 100.00 |

**Claims**

1. A skin care composition comprising:

    i. a medium having a mixture of two or more immiscible phases, wherein the mixture of the two or more immiscible phases comprises (i) 0.1 *wt.%* to 20 *wt.%* of at least one discontinuous internal phase and (ii) 15 *wt.%* to 45 *wt.%* of at least one continuous external phase, wherein the discontinuous and the continuous phases are selected from the group of an oil phase, an aqueous phase, or a silicone phase;
    ii. 0.1 *wt. %* to 10 *wt.%* of a terpolymer of vinyl pyrrolidone/acrylic acid/lauryl methacrylate having a relative viscosity of at least 3.3 cps ( 1cp corresponds to mPa*s, measured according to the capillary viscosimetry method outlined in the description)
    iii. 0.1 *wt. %* to 5 *wt. %* of at least one primary surfactant, co-surfactant or a system of surfactants comprising at least one primary surfactant and at least one co-surfactant; and
    iv. 0.1 *wt. %* to 20 *wt.%* of at least one cosmetically/dermatologically acceptable skin care additive which is selected from the group consisting of diluents, emollients, humectants, thickeners, preservatives, coloring agents, moisturizing agents, film formers/waterproofing agents, bio-active ingredients, pharmaceutical ingredients, pH adjusting agents, chelating agents, rheology modifying agents, fragrance agents, plant extracts, absorbents, vitamins, photo stabilizing agents, sunscreen boosters, anti-oxidants, exfoliating agents, liquid carriers, waxes, conditioners, lubricants, anti-bacterial agents, anti-aging agents, and various combinations thereof.

2. The skin care composition according to claim 1, wherein

    - the weight average molecular weight of terpolymer ranges from 150,000 to 300,000 Daltons; or
    - the primary surfactant, the co-surfactant, and/or the system of surfactant is selected from the group consisting of anionic, cationic, and/or non-ionic surfactants; or
- the composition is a sun screen composition or a cosmetic composition; or
- the composition is in the form of a gel, a balm, a cream-in-gel, a balm-in-gel, a stick, a lotion, a foam, a foam cream, a cream, a moisturizer, a spray, an ointment, and a lamellar gel; or
- the pH of the composition is in the range of from about 2 to about 12; or
- the composition exhibits an increase in contact angle of at least 5° after immersion in water, measured in accordance with the method disclosed in US 2013/0243703.

3. The skin care composition according to claim 1, which is a sunscreen composition comprising:

i. a medium having a mixture of two or more immiscible phases, wherein the mixture of the two or more immiscible phases comprises (i) 0.1 *wt.%* to 20 *wt.%* of at least one discontinuous internal phase and (ii) 15 *wt.%* to 45 *wt.%* of at least one continuous external phase, wherein the discontinuous and the continuous phases are selected from the group of an oil phase, an aqueous phase, or a silicone phase;
ii. 0.1 wt. % to 10 wt.% of a terpolymer of vinyl pyrrolidone/acrylic acid/lauryl methacrylate having a relative viscosity of at least 3.3 *cps*;
iii. 0.1 *wt.* % to 5 *wt.* % of at least one primary surfactant, co-surfactant or a system of surfactants comprising at least one primary surfactant and at least one co-surfactant;
iv. 0.1 *wt.* % to 60 *wt.* % of at least one sunscreen agent; and
v. 0.1 *wt.* % to 20 *wt.%* of at least one cosmetically/dermatologically acceptable skin care additive which is selected from the group consisting of diluents, emollients, humectants, thickeners, preservatives, coloring agents, moisturizing agents, film formers/waterproofing agents, bio-active ingredients, pharmaceutical ingredients, pH adjusting agents, chelating agents, rheology modifying agents, fragrance agents, plant extracts, absorbents, vitamins, photo stabilizing agents, sunscreen boosters, anti-oxidants, exfoliating agents, liquid carriers, waxes, conditioners, lubricants, anti-bacterial agents, anti-aging agents, and various combinations thereof.

4. The sunscreen composition according to claim 3, wherein

- the weight average molecular weight of terpolymer ranges from 150,000 to 300,000 Daltons; or
- the primary surfactant, the co-surfactant, and/or the system of surfactant is selected from the group consisting of anionic, cationic, and/or non-ionic surfactants; or
- the sunscreen agent is selected from the group consisting of methoxydibenzoylmethane; octyl salicylate; pentyl dimethyl PABA; octyl dimethyl PABA; benzophenone-1; benzophenone-6; 2-(2H-benzotriazole-2-yl)-4,6-di-tert-pentylphenol; ethyl-2-cyano-3,3-diphenylacrylate; homomethyl salicylate (homosalate); bis-ethylhexyloxyphenol methoxyphenyl triazine; methyl-(1,2,2,6,6-pentamethyl-4- piperidyl)-sebacate; 2-(2H-benzotriazole-2-yl)-4-methylphenol; diethylhexyl butamido triazone; amyl dimethyl PABA; 4,6-bis(octylthiomethyl)-o-cresol; red petroleum; ethylhexyl triazone; octocrylene; isoamyl-p-methoxycinnamate; drometrizole; titanium dioxide; 2,4-di-tert-butyl-6-(5-chloro-2H-benzotriazole-2-yl)-phenol; 2-hydroxy-4- octyloxybenzophenone; benzophenone-2; diisopropyl methylcinnamate; PEG-25 PABA; 2-(1,1-dimethylethyl)-6-[[3-(1,1-demethylethyl)-2-hydroxy-5-methylphenyl]methyl-4- methylphenyl acrylate; drometrizole trisiloxane; menthyl anthranilate; butyl methoxydibenzoylmethane; 2-ethoxyethyl p-methoxycinnamate; benzylidene camphor sulfonic acid; dimethoxyphenyl-[1-(3,4)]-4,4-dimethyl 1,3-pentanedione; zinc oxide; N,N'-hexane-1,6-diylbis[3-(3,5-di-tert-butyl-4-hydroxyphenylpropionamide)]; pentaerythritol tetrakis[3-(3,5-di-tert-butyl-4-hydroxyphenyl)propionate] ; 2,6-di-tert- butyl-4-[4,6-bis(octylthio)-1,3,5-triazin-2-ylamino]phenol; 2-(2H-benzotriazole-2-yl)- 4,6-bis(1-methyl-1-phenyl-lethyl)phenol; trolamine salicylate; diethylanolamine p-methoxycinnamate; polysilicone-15; 4-methylbenzylidene camphor; bisoctrizole; N-phenyl-benzenamine; reaction products with 2,4,4-trimethylpentene; sulisobenzone; (2- ethylhexyl)-2-cyano-3,3-diphenylacrylate; digalloyl trioleate; polyacrylamido methylbenzylidene camphor; glyceryl ethylhexanoate dimethoxycinnamate; 1,3-bis-[(2'- cyano-3',3'-diphenylacryloyl)oxy]-2,2-bis-[(2'-cyano-bis-(2,2,6,6-tetramethyl-4-piperidyl) - sebacate ; benzophenone- 5; 1 , 3 , 5 -tris (3 ,5 -di-tert-butyl-4-hydroxybenzyl) -1,3,5- triazine-2,4,6(1H,3H,5H)-trione; hexamethylendiamine; benzophenone- 8; ethyl-4- bis(hydroxypropyl)aminobenzoate; 6-tert-butyl-2-(5-chloro-2H-benzotriazole-2-yl)-4- methylphenol; p-aminobenzoic acid; 3,3',3'',5,5',5''-hexa-tert-butyl-a-a'-a''-(mesitylene- 2,4,6-triyl)tri-p-cresol; lawsone with dihydroxyacetone; benzophenone-9; benzophenone - 4; ethylhexyl dimethoxy benzylidene dioxoimidazoline propionate; N,N'-bisformyl-N,N'- bis-(2,2,6,6-tetramethyl-4-piperidinyl)-; 3-benzylidene camphor; terephthalylidene dicamphor sulfonic acid; camphor benzalkonium methosulfate; bisdisulizole disodium; etocrylene; ferulic acid; 2-(2H-benzotriazole-2-yl)-4-(l,l,3,3-tetramethylbutyl)-phenol; 4,6-bis(dodecylthiomethyl)-o-cresol; β-2-glucopyranoxy propyl hydroxy benzophenone; phenylbenzimidazole sulfonic acid; benzophenone- 3; diethylamine hydroxybenzoyl hexylbenzoate; 3',3'-diphenylacryloyl)oxy]methyl}-propane; ethylhexyl p- methoxycinnamate, and blends thereof; or
- the composition is in the form of a gel, a balm, a cream-in-gel, a balm-in-gel, a stick, a lotion, a foam, a foam cream, a cream, a moisturizer, a spray, an ointment, and a lamellar gel; or
- the pH of the composition is in the range of from about 2 to about 12.

5. The sunscreen composition accordingly to claim 3, wherein the composition exhibits an increase in contact angle of at least 5° after immersion in water, measured in accordance with the method disclosed in US 2013/0243703.

**Patentansprüche**

1. Eine Hautpflegezusammensetzung umfassend:

i. ein Medium mit einer Mischung aus zwei oder mehr nicht mischbaren Phasen, wobei die Mischung aus den zwei oder mehr nicht mischbaren Phasen (i) 0,1 *Gew.-%* bis 20 *Gew.*-% mindestens einer diskontinuierlichen inneren Phase und (ii) 15 *Gew.-%* bis 45 *Gew.-%* mindestens einer kontinuierlichen äußeren Phase umfasst, wobei die diskontinuierliche und die kontinuierliche Phase aus der Gruppe einer Ölphase, einer wässrigen Phase oder einer Silikonphase ausgewählt sind;

ii. 0,1 *Gew.-%* bis 10 *Gew.*-% eines Terpolymers aus Vinylpyrrolidon/Acrylsäure/Laurylmethacrylat mit einer relativen Viskosität von mindestens 3,3 *cps* (1 *cp* entspricht mPa*s der Kapillarviskosimeter-Methode gemäß der Beschreibung);

iii. 0,1 *Gew.-%* bis 5 *Gew.-%* mindestens eines primären Tensids, Co-Tensids oder eines Tensidsystems, das mindestens ein primäres Tensid und mindestens ein Co-Tensid umfasst; und

iv. 0,1 *Gew.-%* bis 20 *Gew.*-% mindestens eines kosmetisch/dermatologisch akzeptablen Hautpflegezusatzstoffs, der aus der Gruppe ausgewählt ist, die aus Verdünnungsmitteln, Weichmachern, Feuchthaltemitteln, Verdickungsmitteln, Konservierungsmitteln, Farbstoffen, Feuchtigkeitsmitteln, Filmbildnern/Wasserabweisungsmitteln, bioaktiven Inhaltsstoffen, pharmazeutischen Inhaltsstoffen, pH-Regulatoren, Chelatbildnern, Rheologiemodifikatoren, Duftstoffen, Pflanzenextrakten, Absorptionsmitteln, Vitaminen, Lichtschutzmitteln, Sonnenschutzverstärkern, Antioxidantien, Peelingmitteln, flüssigen Trägern, Wachsen, Konditionierungsmitteln, Gleitmitteln, antibakteriellen Mitteln, Anti-Aging-Mitteln und verschiedenen Kombinationen davon besteht.

2. Die Hautpflegezusammensetzung gemäß Anspruch 1, wobei

- das gewichtsmittlere Molekulargewicht des Terpolymers im Bereich von 150.000 bis 300.000 Dalton liegt; oder
- das primäre Tensid, das Co-Tensid und/oder das Tensidsystem aus der Gruppe ausgewählt ist, die aus anionischen, kationischen und/oder nichtionischen Tensiden besteht; oder
- die Zusammensetzung eine Sonnenschutzzusammensetzung oder eine kosmetische Zusammensetzung ist; oder
- die Zusammensetzung in Form eines Gels, eines Balsams, einer Creme-in-Gel, eines Balsam-in-Gel, eines Stifts, einer Lotion, eines Schaums, einer Schaumcreme, einer Creme, einer Feuchtigkeitscreme, eines Sprays, einer Salbe und eines lamellaren Gels vorliegt; oder
- der pH-Wert der Zusammensetzung im Bereich von etwa 2 bis etwa 12 liegt; oder
- die Zusammensetzung nach dem Eintauchen in Wasser eine Zunahme des Kontaktwinkels von mindestens 5° aufweist, gemessen gemäß dem in US 2013/0243703 offenbarten Verfahren.

3. Die Hautpflegezusammensetzung gemäß Anspruch 1, die eine Sonnenschutzzusammensetzung ist, umfassend:

i. ein Medium mit einer Mischung aus zwei oder mehr nicht mischbaren Phasen, wobei die Mischung aus den zwei oder mehr nicht mischbaren Phasen (i) 0,1 *Gew.-%* bis 20 *Gew.*-% mindestens einer diskontinuierlichen inneren Phase und (ii) 15 *Gew.-%* bis 45 *Gew.-%* mindestens einer kontinuierlichen äußeren Phase umfasst, wobei die diskontinuierliche und die kontinuierliche Phase aus der Gruppe einer Ölphase, einer wässrigen Phase oder einer Silikonphase ausgewählt sind;

ii. 0,1 Gew.-% bis 10 Gew.-% eines Terpolymers aus Vinylpyrrolidon/Acrylsäure/Laurylmethacrylat mit einer relativen Viskosität von mindestens 3,3 *cps*;

iii. 0,1 *Gew.-%* bis 5 *Gew.-%* mindestens eines primären Tensids, Co-Tensids oder eines Tensidsystems, das mindestens ein primäres Tensid und mindestens ein Co-Tensid umfasst;

iv. 0,1 *Gew.-%* bis 60 *Gew.-%* mindestens eines Sonnenschutzmittels; und

v. 0,1 *Gew.-%* bis 20 *Gew.*-% mindestens eines kosmetisch/dermatologisch akzeptablen Hautpflegezusatzstoffes, der aus der Gruppe ausgewählt ist, die aus Verdünnungsmitteln, Weichmachern, Feuchthaltemitteln, Verdickungsmitteln, Konservierungsmitteln, Farbstoffen, Feuchtigkeitsmitteln, Filmbildnern/Wasserabweisungsmitteln, bioaktiven Inhaltsstoffen, pharmazeutischen Inhaltsstoffen, pH-Regulatoren, Chelatbildnern, Rheologiemodifikatoren, Duftstoffen, Pflanzenextrakten, Absorptionsmitteln, Vitaminen, Lichtstabilisatoren, Sonnenschutzverstärkern, Antioxidantien, Peelingmitteln, flüssigen Trägern, Wachsen, Konditionierungsmitteln, Gleitmitteln, antibakteriellen Mitteln, Anti-Aging-Mitteln und verschiedenen Kombinationen davon besteht.

4. Die Sonnenschutzzusammensetzung gemäß Anspruch 3, wobei

- das gewichtsmittlere Molekulargewicht des Terpolymers im Bereich von 150.000 bis 300.000 Dalton liegt; oder
- das primäre Tensid, das Co-Tensid und/oder das Tensidsystem aus der Gruppe ausgewählt ist, die aus anionischen, kationischen und/oder nichtionischen Tensiden besteht; oder
- der Sonnenschutzwirkstoff aus der Gruppe ausgewählt ist, die aus Methoxydibenzoylmethan, Octylsalicylat, Pentyl-Dimethyl-PABA, Octyl-Dimethyl-PABA, Benzophenon-1, Benzophenon-6, 2-(2H-Benzotriazol-2-yl)-4,6-di-tert-pentylphenol, Ethyl-2-cyano-3,3-diphenylacrylat; Homomethylsalicylat (Homosalat); Bis-ethylhexyloxyphenolmethoxyphenyltriazin; Methyl-(1,2,2,6,6-pentamethyl-4-piperidyl)-sebacat; 2-(2H-Benzotriazol-2-yl)-4-methylphenol; Diethylhexylbutamidotriazon; Amyldimethyl-PABA; 4,6-Bis(octylthiomethyl)-o-kresol; rotes Petroleum; Ethylhexyl-Triazon; Octocrylen; Isoamyl-p-Methoxycinnamat; Drometrizol; Titandioxid; 2,4-Di-tert-Butyl-6-(5-Chlor-2H-benzotriazol-2-yl)-phenol; 2-Hydroxy-4-octyloxybenzophenon; Benzophenon-2; Diisopropylmethylcinnamat; PEG-25 PABA; 2-(1,1-Dimethylethyl)-6-[[3-(1,1-Dimethylethyl)-2-hydroxy-5-methylphenyl]methyl-4-methylphenylacrylat; Drometrizol-Trisiloxan; Menthylanthranilat; Butylmethoxydibenzoylmethan; 2-Ethoxyethyl-p-methoxycinnamat; Benzylidenkampfersulfonsäure; Dimethoxyphenyl-[l-(3,4)]-4,4-dimethyl-1,3-pentandion; Zinkoxid; N,N'-Hexan-1,6-diylbis[3-(3,5-di-tert-butyl-4-hydroxyphenylpropionamid)]; Pentaerythritoltetrakis[3-(3,5-di-tert-butyl-4-hydroxyphenyl)propionat]; 2,6-Di-tert-butyl-4-[4,6-bis(octylthio)-l,3,5-triazin-2-ylamino]phenol; 2-(2H-Benzotriazol-2-yl)-4,6-bis(1-methyl-1-phenylethyl)phenol; Trolaminsalicylat; Diethanolamin-p-methoxycinnamat; Polysilikon-15; 4-Methylbenzylidenkampfer; Bisoctrizol; N-Phenylbenzenamin; Reaktionsprodukte mit 2,4,4-Trimethylpenten; Sulisobenzon; (2-Ethylhexyl)-2-cyano-3,3-diphenylacrylat; Digalloyltrioleat; Polyacrylamidomethylbenzylidene-Campher; Glycerylethylhexanoatdimethoxycinnamat; 1,3-Bis-[(2'-cyano-3',3'-diphenylacryloyl)oxy]-2,2-bis-[(2'-cyano-bis-(2,2,6,6-tetramethyl-4-piperidyl)-sebacat; Benzophenon-5; 1,3,5-Tris(3,5-di-tert-butyl-4-hydroxybenzyl)-1,3,5-triazin-2,4,6(1H,3H,5H)-trion; Hexamethylendiamin; Benzophenon-8; Ethyl-4-bis(hydroxypropyl)aminobenzoat; 6-tert-Butyl-2-(5-chlor-2H-benzotriazol-2-yl)-4-methylphenol; p-Aminobenzoesäure; 3,3',3'',5,5',5''-Hexa-tert-butyl-a-a'-a''-(mesitylen-2,4,6-triyl)tri-p-kresol; Lawson mit Dihydroxyaceton; Benzophenon-9; Benzophenon-4; Ethylhexyl-dimethoxybenzylidendioxoimidazolinpropionat; N,N'-Bisformyl-N,N'-bis-(2,2,6,6-tetramethyl-4-piperidinyl)-; 3-Benzylidenkampfer; Terephthalylidendikampfersulfonsäure; Kampferbenzalkoniummethosulfat; Bisdisulizoldinatriumsalz; Etocrylen; Ferulasäure; 2-(2H-Benzotriazol-2-yl)-4-(1,1,3,3-tetramethylbutyl)-phenol; 4,6-Bis(dodecylthiomethyl)-o-kresol; β-2-Glucopyranoxypropylhydroxybenzophenon; Phenylbenzimidazolsulfonsäure; Benzophenon-3; Diethylaminhydroxybenzoylhexylbenzoat; 3',3'-Diphenylacryloyloxy]methyl}-propan; Ethylhexyl-p-methoxycinnamat und Mischungen davon besteht; oder
- die Zusammensetzung in Form eines Gels, eines Balsams, einer Creme-in-Gel, eines Balsams-in-Gel, eines Stifts, einer Lotion, eines Schaums, einer Schaumcreme, einer Creme, einer Feuchtigkeitscreme, eines Sprays, einer Salbe und eines lamellaren Gels vorliegt; oder
- der pH-Wert der Zusammensetzung im Bereich von etwa 2 bis etwa 12 liegt.

5. Die Sonnenschutzzusammensetzung gemäß Anspruch 3, wobei die Zusammensetzung nach dem Eintauchen in Wasser eine Erhöhung des Kontaktwinkels um mindestens 5° aufweist, gemessen gemäß dem in US 2013/0243703 offenbarten Verfahren.

**Revendications**

1. Composition de soin pour la peau comprenant :

i. un milieu contenant un mélange de deux ou plusieurs phases non miscibles, dans lequel le mélange des deux ou plusieurs phases non miscibles comprend (i) 0,1 *% en poids* à 20 *% en poids* d'au moins une phase interne discontinue et (ii) 15 *% en poids* à 45 *% en poids* d'au moins une phase externe continue, dans lequel les phases discontinue et continue sont choisies dans le groupe constitué d'une phase huileuse, d'une phase aqueuse ou d'une phase silicone;

ii. 0,1 % à 10 *% en poids* d'un terpolymère de vinylpyrrolidone/acide acrylique/méthacrylate de lauryle ayant une viscosité relative d'au moins 3,3 *cps* (1 *cp* correspond à mPa*s selon la méthode du viscosimètre capillaire telle que décrite);

iii. 0,1 % à 5 % *en poids* d'au moins un tensioactif primaire, un co-tensioactif ou un système de tensioactifs comprenant au moins un tensioactif primaire et au moins un co-tensioactif; et

iv. 0,1 % à 20 *% en poids* d'au moins un additif de soin de la peau acceptable sur le plan cosmétique/dermatologique, choisi dans le groupe constitué par les diluants, les émollients, les humectants, les épaississants, les conservateurs, les colorants, les agents hydratants, les agents filmogènes/imperméabilisants, les ingrédients bioactifs, les ingrédients pharmaceutiques, agents ajustant le pH, agents chélateurs, agents modifiant la rhéologie, agents parfumants, extraits végétaux, absorbants, vitamines, agents photostabilisants, agents renforçant l'efficacité des écrans solaires, antioxydants, agents exfoliants, véhicules liquides, cires, agents conditionneurs, lubrifiants, agents antibactériens, agents anti-vieillissement et diverses combinaisons de ceux-ci.

2. Composition de soin de la peau selon la revendication 1, dans laquelle

- le poids moléculaire moyen en poids du terpolymère est compris entre 150 000 et 300 000 daltons; ou
- le tensioactif primaire, le co-tensioactif et/ou le système tensioactif sont choisis dans le groupe constitué par les tensioactifs anioniques, cationiques et/ou non ioniques; ou
- la composition est une composition de protection solaire ou une composition cosmétique; ou
- la composition se présente sous la forme d'un gel, d'un baume, d'une crème-gel, d'un baume-gel, d'un stick, d'une lotion, d'une mousse, d'une crème mousse, d'une crème, d'un hydratant, d'un spray, d'une pommade et d'un gel lamellaire; ou
- le pH de la composition est compris entre environ 2 et environ 12; ou
- la composition présente une augmentation de l'angle de contact d'au moins 5° après immersion dans l'eau, mesurée conformément à la méthode décrite dans le document US 2013/0243703.

3. Composition de soin de la peau selon la revendication 1, qui est une composition de protection solaire comprenant:

i. un milieu ayant un mélange de deux ou plusieurs phases non miscibles, dans lequel le mélange des deux ou plusieurs phases non miscibles comprend (i) 0,1 *% en poids* à 20 *% en poids* d'au moins une phase interne discontinue et (ii) 15 *% en poids* à 45 *% en poids* d'au moins une phase externe continue, dans laquelle les phases discontinue et continue sont choisies dans le groupe constitué d'une phase huileuse, d'une phase aqueuse ou d'une phase silicone;

ii. 0,1 % à 10 % en poids d'un terpolymère de vinylpyrrolidone/acide acrylique/méthacrylate de lauryle ayant une viscosité relative d'au moins 3,3 *cps*;

iii. 0,1 % à 5 % *en poids* d'au moins un tensioactif primaire, un co-tensioactif ou un système de tensioactifs comprenant au moins un tensioactif primaire et au moins un co-tensioactif;

iv. 0,1 % à 60 *% en poids* d'au moins un agent écran solaire; et

v. 0,1 % à 20 *% en poids* d'au moins un additif cosmétique/dermatologique acceptable pour les soins de la peau, choisi parmi le groupe comprenant les diluants, les émollients, les humectants, les épaississants, les conservateurs, les colorants, les agents hydratants, les agents filmogènes/imperméabilisants, les ingrédients bioactifs, les ingrédients pharmaceutiques, agents ajustant le pH, agents chélateurs, agents modifiant la rhéologie, agents parfumants, extraits végétaux, absorbants, vitamines, agents photostabilisants, agents renforçant l'efficacité des écrans solaires, antioxydants, agents exfoliants, véhicules liquides, cires, agents revitalisants, lubrifiants, agents antibactériens, agents anti-vieillissement et diverses combinaisons de ceux-ci.

4. Composition de protection solaire selon la revendication 3, dans laquelle

- le poids moléculaire moyen en poids du terpolymère est compris entre 150 000 et 300 000 Daltons; ou
- le tensioactif primaire, le co-tensioactif et/ou le système de tensioactifs est choisi dans le groupe constitué des tensioactifs anioniques, cationiques et/ou non ioniques; ou
- l'agent photoprotecteur est choisi parmi le groupe constitué du méthoxydibenzoylméthane; du salicylate d'octyle; du pentyl diméthyl PABA; de l'octyl diméthyl PABA; de la benzophénone-1; de la benzophénone-6; du 2-(2H-benzotriazole-2-yl)-4,6-di-tert-pentylphénol; acrylate d'éthyle-2-cyano-3,3-diphényle; salicylate d'homométhyle (homosalate); bis-éthylhexyloxyphénol méthoxyphényl triazine; méthyl-(1,2,2,6,6-pentaméthyl-4-pipéridyl)-sébacate; 2-(2H-benzotriazole-2-yl)-4-méthylphénol; diéthylhexyl butamido triazone; amyl diméthyl PABA; 4,6-bis(octylthiométhyl)-o-crésol; pétrole rouge; éthylhexyl triazone; octocrylène; isoamyl-p-méthoxycinnamate; drométrizole; dioxyde de titane; 2,4-di-tert-butyl-6-(5-chloro-2H-benzotriazole-2-yl)-phénol; 2-hydroxy-4-octyloxybenzophénone; benzophénone-2; diisopropyl méthylcinnamate; PEG-25 PABA; 2-(l,l-diméthyléthyl)-6-[[3-(l,l-déméthyléthyl)-2-hydroxy-5-méthylphényl]méthyl-4-méthylphényl acrylate; drométrizole trisiloxane; anthranilate de menthyle; butyl méthoxydibenzoylméthane; 2-éthoxyéthyl p-méthoxycinnamate; acide benzylidène camphor sulfonique; diméthoxyphényl-[l-(3,4)]-4,4-diméthyl 1,3-pentanedione; oxyde de zinc; N,N'-hexane-l,6-diylbis[3-(3,5-di-tert-butyl-4-hydroxyphénylpropionamide)]; pentaérythritol tétrakis[3-(3,5-di-tert-butyl-4-hydroxyphényl)propionate]; 2,6-di-tert-butyl-4-[4,6-bis(octylthio)-l,3,5-triazine-2-ylamino]phénol; 2-(2H-benzotriazole-2-yl)-4,6-bis(1-méthyl-1-phényléthyl)phénol; salicylate de trolamine; p-méthoxycinnamate de diéthylanolamine; polysilicone-15; 4-méthylbenzylidène camphre; bisoctrizole; N-phényl-benzénamine; produits de réaction avec le 2,4,4-triméthylpentène; sulisobenzone; (2-éthylhexyl)-2-cyano-3,3-diphénylacrylate; trioléate de digalloyle; polyacrylamido méthylbenzylidène camphre; diméthoxycinnamate de glycéryle éthylhexanoate; 1,3-bis-[(2'-cyano-3',3'-diphénylacryloyl)oxy]-2,2-bis-[(2'-cyano-bis-(2,2,6,6-tétraméthyl-4-pipéridyl) - sébacate; benzophénone-5; 1,3,5-tris(3,5-di-tert-butyl-4-hydroxybenzyl)-1,3,5-triazine-2,4,6(1H,3H,5H)-trione; hexaméthylènediamine; benzophénone-8; éthyl-4-bis(hydroxypropyl)aminobenzoate; 6-tert-butyl-2-(5-chloro-2H-benzotriazole-2-yl)-4-méthylphénol; acide p-aminobenzoïque; 3,3',3",5,5',5"-hexa-tert-butyl-a-a'-a"-(mésitylène-2,4,6-triyl)tri-p-crésol; lawsone avec dihydroxyacétone; benzophénone-9; benzophénone-4; propionate d'éthylhexyl diméthoxy benzylidène dioxoimidazoline; N,N'-bisformyl-N,N'-bis-(2,2,6,6-tétraméthyl-4-pipéridinyl)-; 3-benzylidène camphre; acide téréphtalylidène dicamphre sulfonique; méthosulfate de camphre benzalkonium; bisdisulizole disodique; étocrylène; acide férulique; 2-(2H-benzotriazole-2-yl)-4-(1,l,3,3-tétraméthylbutyl)-phénol; 4,6-bis(dodécylthiométhyl)-o-crésol; β-2-glucopyranoxy propyl hydroxy benzophénone; acide phénylbenzimidazole sulfonique; benzophénone-3; diéthylamine hydroxybenzoyl hexylbenzoate; 3',3'-diphénylacryloyl)oxy]méthyl}-propane; éthylhexyl p-méthoxycinnamate, et leurs mélanges; ou
- la composition se présente sous la forme d'un gel, d'un baume, d'une crème-gel, d'un baume-gel, d'un stick, d'une lotion, d'une mousse, d'une crème mousse, d'une crème, d'un hydratant, d'un spray, d'une pommade et d'un gel lamellaire; ou
- le pH de la composition est compris entre environ 2 et environ 12.

5. Composition de protection solaire selon la revendication 3, dans laquelle la composition présente une augmentation de l'angle de contact d'au moins 5° après immersion dans l'eau, mesurée selon la méthode décrite dans le document US 2013/0243703.

Water resistance study of the skin care compositions of Examples 1a, 1b and 1c

**FIG. 1**

Water resistance study of the skin care compositions of Examples 3a, 3b and 3c

**FIG. 2**

**Water resistance study of the skin care compositions of Examples 4a and 4b**

**FIG. 3**

**Water resistance study of the skin care compositions of Examples 6a and 6b**

**FIG. 4**

**Water resistance study of the skin care compositions of Examples 7a and 7b**

**FIG. 5**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2012195839 A
- US 2009023662 W
- US 6294158 B
- WO 2009023662 A2
- WO 2006097514 A1
- US 20130243703 A

### Non-patent literature cited in the description

- *McCutcheon's Detergents and Emulsifiers*, 1982
- **KIRK-OTHMER**. *Encyclopedia of Chemical Technology*, vol. 22, 346-387
- **P. D. DORGAN**. *Drug and Cosmetic Industry*, December 1983, 30-33
- **KIRK-OTHMER**. *Encyclopedia of Chemical Technology*, vol. 5, 857-884
- **T. E. FURIA**. *CRC Handbook of Food Additives*, 1972, 271-294
- Encyclopedia of Food Science. 1978, 694-699